# EUROPEAN PATENT SPECIFICATION

(11) **EP 4 452 168 B1**
(45) Date of publication and mention of the grant of the patent: **01.04.2026**
(21) Application number: 22760820.5
(22) Date of filing: 29.06.2022
(51) Int. Cl.: A61G 5/06, A61F 5/37, A61G 5/10

(54) **PATIENT CONTAINMENT SYSTEMS FOR USE WITH PATIENT TRANSPORT APPARATUSES**
PATIENTENBEHÄLTERSYSTEME ZUR VERWENDUNG MIT PATIENTENTRANSPORTVORRICHTUNGEN
SYSTÈMES DE CONFINEMENT DE PATIENT DESTINÉS À ÊTRE UTILISÉS AVEC DES APPAREILS DE TRANSPORT DE PATIENT

(30) Priority: 22.12.2021 US 202163292497 P
(43) Date of publication of application: 30.10.2024
(73) Proprietor: Stryker Corporation, Portage, MI 49002-9711 (US)
(72) Inventor: TUMAVICH, James, Robert, Kalamazoo, MI 49007 (US); MATHENY, Nathan, W., Portage, MI 49024 (US); HERBST, Cory, P., Shelbyville, MI 49344 (US); TESSMER, Brian, J., Mattawan, MI 49071 (US)
(74) Representative: Röthinger, Rainer
(86) International application number: PCT/US2022/035498
(87) International publication number: WO 2023/121716

(56) References cited:
- EP-B1- 3 400 921
- US-A1- 2021 196 535

## Description

### CROSS-REFERENCE TO RELATED APPLICATIONS

The subject patent application claims priority to, and all the benefits of, United States Provisional Patent Application No. 63/292,497, filed on December 22, 2021.

### BACKGROUND

In many instances, patients with limited mobility may have difficulty traversing stairs without assistance. In certain emergency situations, traversing stairs may be the only viable option for exiting a building. In order for a caregiver to transport a patient along stairs in a safe and controlled manner, a stair chair or evacuation chair may be utilized. Stair chairs are adapted to transport seated patients either up or down stairs, with two caregivers typically supporting, stabilizing, or otherwise carrying the stair chair with the patient supported thereon.

Typically, caregivers will secure the patient to the stair chair using an arrangement of straps, harnesses, and the like, which may be adjustable to conform to different patient body types. As such, these conventional straps tend to be of excess length to allow usage with patients of a variety of body types. However, when these conventional straps are used with certain types of patients (e.g., shorter or younger patients), they can include an excess length and be difficult to manage, especially while operating the stair chair. EP 3 400 921 B1 discusses a locking device of a patient transport device for locking at least one belt device of a patient restraint device on the patient transport device.

A patient containment system that addresses one or more of the aforementioned challenges is desired.

### SUMMARY

Independent claim 1, to which the reader should now refer, concerns a patient transport system. The dependent claims concern specific embodiments thereof. According to an example, a patient transport system including a patient transport apparatus with a seat section and a back section for supporting the patient; and a patient containment system including a lower strap, an upper strap having a front end coupled to the lower strap and a back end, and a coupling system to facilitate releasable attachment of the patient containment system to the patient transport apparatus. The coupling system includes a latch coupled to the back end of the upper strap, a coupling bracket configured for removable attachment to the back section of the patient transport apparatus, and a keeper operatively attached to the coupling bracket and configured to removably receive the latch to retain the upper strap together with the latch relative to the coupling bracket.

### BRIEF DESCRIPTION OF THE DRAWINGS

Other advantages of the present invention will be readily appreciated as the same becomes better understood by reference to the following detailed description when considered in connection with the accompanying drawings.
Figure 1 is a front perspective view of a patient transport apparatus and a front perspective view of a patient containment system according to the present disclosure.
Figure 2 is a partial view of the patient transport apparatus of Figure 1 and a perspective view of the patient containment system Figure 1.
Figure 3 is a rear perspective view of the patient transport apparatus of Figures 1-2 and a rear perspective view of the patient containment system Figures 1-2.
Figure 4 is a bottom perspective view of the patient transport apparatus of Figures 1-3 and a rear perspective view of the patient containment system Figures 1-3.
Figure 5A is a front view of a coupling bracket and a keeper of the patient containment system of Figures 1-4.
Figure 5B is a top view of the coupling bracket and the keeper of Figure 5A.
Figure 5C is a cutaway view of the coupling bracket and the keeper of Figures 5A-5B.
Figure 6 is a front view of a latch of the patient containment system Figures 1-4.
Figure 7A is a front perspective view of an upper strap lock of the patient containment system of Figures 1-4.
Figure 7B is a cutaway view of the upper strap lock of Figure 7A
Figure 8A is a perspective view of the upper strap lock of Figures 7A and 7B shown operating in a released configuration.
Figure 8B is a perspective view of the upper strap lock of Figure 8A shown operating in a retained configuration.
Figure 9A is a front perspective view of an anchor of the patient containment system of Figures 1-4.
Figure 9B is a side view of the anchor of Figure 9A.
Figure 10A is a front perspective of a coupling system of the patient containment system of Figures 1-4 shown operating in a disengaged configuration.
Figure 10B is a side cutaway view of the coupling system of Figure 10A shown operating in the disengaged configuration.
Figure 11A is a front perspective view of the coupling system of Figure 10A shown operating in a transitional configuration.
Figure 11B is a side cutaway view of the coupling system of Figure 11A shown operating in the transitional configuration.
Figure 12A is a front perspective view of the coupling system of Figure 10A shown operating in an engaged configuration.
Figure 12B is a side cutaway view of the coupling system of Figure 12A shown operating in the engaged configuration.
Figure 13 is a front perspective view of the coupling system of Figures 10A-12B shown operating in the engaged configuration and with the upper strap lock of Figures 8A-8B shown operating in the retained configuration.
Figure 14 is a front perspective view of a seat section coupling bracket of the patient containment system of Figures 1-4.
Figure 15A is a front perspective view depicting a latch with a blocking tab.
Figure 15B is a front perspective view of a coupling system including the latch of Figure 15A and shown operating in an engaged configuration.
Figures 16A and 16B are partial perspective views of the patient containment system of Figures 1-4 including the seat section coupling bracket of Figure 14 and the coupling bracket of Figures 5A-5C.

### DETAILED DESCRIPTION

Referring now to the drawings, wherein like numerals indicate like parts throughout the several views, the present disclosure is generally directed toward a patient transport apparatus 100 configured to allow one or more caregivers to transport a patient. In some versions, the patient transport apparatus 100 may be similar to as is disclosed in U.S. Patent Application Publication No. 2021/0196535, entitled "Patient Containment Systems For Use With Patient Transport Apparatuses."

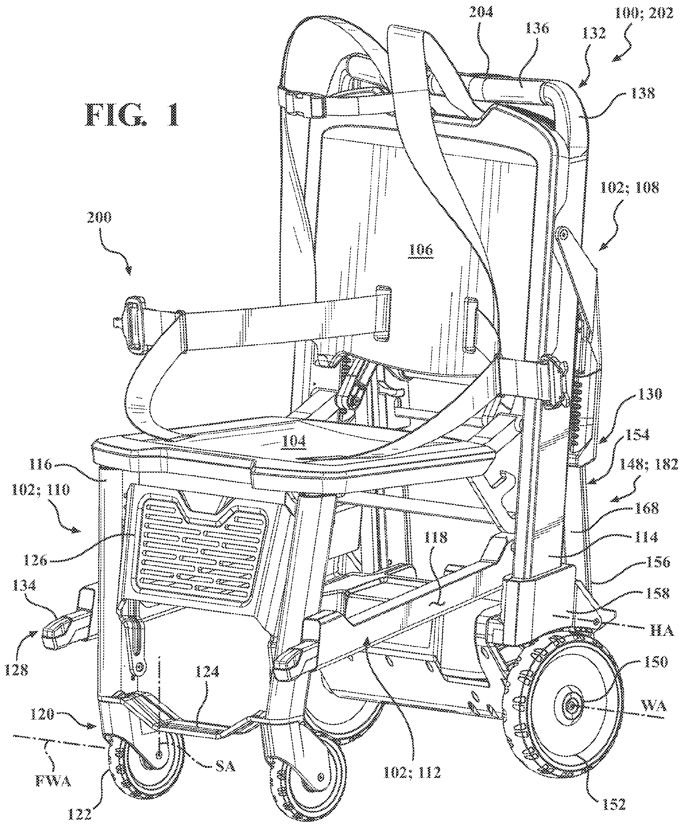

The patient transport apparatus 100 illustrated throughout the drawings is realized as a "stair chair" which can be operated in a chair configuration to transport the patient across ground or floor surfaces (e.g., pavement, hallways, and the like) and/or during ingress into or egress out of a structure (e.g., a home or building) as shown in Figure 1, as well as in a stair configuration and/or a stowed configuration (not shown in detail herein) as disclosed in U.S. Patent Application Publication No. 2021/0196535, previously referenced. As is best shown in Figure 1, the patient transport apparatus 100 comprises a support structure 102 to which a seat section 104 and a back section 106 are operatively attached. The seat section 104 and the back section 106 are each shaped and arranged to provide support to the patient during transport. The support structure 102 generally includes a rear support assembly 108, a front support assembly 110, and an intermediate support assembly 112. The back section 106 is coupled to the rear support assembly 108 for concurrent movement. To this end, the rear support assembly 108 comprises rear uprights 114 which extend generally vertically and are secured to the back section 106, such as with fasteners (not shown in detail). The rear uprights 114 are spaced generally laterally from each other in the illustrated versions and are formed from separate components which cooperate to generally define the rear support assembly 108. However, those having ordinary skill in the art will appreciate that other configurations are contemplated, and the rear support assembly 108 could comprise or otherwise be defined by any suitable number of components. The front support assembly 110 comprises front struts 116 which, like the rear uprights 114, are spaced laterally from each other and extend generally vertically. The intermediate support assembly 112 comprises intermediate arms 118 which are also spaced laterally from each other. Here, too, it will be appreciated that other configurations are contemplated, and the front support assembly 110 and/or the intermediate support assembly 112 could comprise or otherwise be defined by any suitable number of components.

In some instances, the intermediate support assembly 112 and the seat section 104 each may be pivotably coupled to the rear support assembly 108. Additionally, the intermediate support assembly 112 and the seat section 104 each may be pivotably coupled to the front support assembly 110, such as is disclosed in U.S. Patent Application Publication No. 2021/0196535, previously referenced. Furthermore, for each of the pivotable connections disclosed herein, it will be appreciated that one or more fasteners, bushings, bearings, washers, spacers, and the like may be provided to facilitate smooth pivoting motion between various components.

Referring to Figure 1, the front support assembly 110 includes a pair of caster assemblies 120, each of which comprises a front wheel 122 arranged to rotate about a respective front wheel axis FWA and to pivot about a respective swivel axis SA (axes not shown in detail). The caster assemblies 120 are generally arranged on opposing lateral sides of the front support assembly 110 and are operatively attached to the front struts 116. A lateral brace 124 extends laterally between the front struts 116 to, among other things, afford rigidity to the support structure 102. Here, a foot rest 126 is pivotably coupled to each of the front struts 116 adjacent to the caster assemblies 120 to provide support to the patient's feet during transport.

The representative versions of the patient transport apparatus 100 illustrated throughout the drawings comprise different handles arranged for engagement by caregivers during patient transport. More specifically, the patient transport apparatus 100 comprises front handle assemblies 128, pivoting handle assemblies 130, and an upper handle assembly 132 (hereinafter referred to as "handle assembly 132"), as described in greater detail below. A stowed position and an engagement position of the pivoting handle assemblies 130 and the handle assembly 132 are further described and shown in U.S. Patent Application Publication No. 2021/0196535, previously referenced.

The front handle assemblies 128 are configured to be operated in a collapsed position and an extended position (not shown). The front handle assemblies 128 are shown in the collapsed position in Figure 1. The front handle assemblies 128 may be slidably supported by bushings, bearings, and the like coupled to the intermediate arms 118, and may be lockable in and/or between the collapsed position and the extended position via respective front handle locks 134 shown in Figure 1. A caregiver may engage the front handle locks 134 (not shown in detail) to facilitate moving the front handle assemblies 128 between the collapsed position and the extended position. The front handle assemblies 128 are generally arranged so as to be engaged by a caregiver during patient transport up or down stairs when in the extended position. It will be appreciated that the front handle assemblies 128 could be of various types, styles, and/or configurations suitable to be engaged by caregivers to support the patient transport apparatus 100 for movement. While the illustrated front handle assemblies 128 are arranged for telescoping movement, other configurations are contemplated. By way of non-limiting example, the front handle assemblies 128 could be pivotably coupled to the support structure 102 or other parts of the patient transport apparatus 100. In some versions, the front handle assemblies 128 could be configured in a manner similar to that disclosed in U.S. Patent No. 6,648,343.

The pivoting handle assemblies 130 are configured to be operated in a stowed position and an engagement position (not shown). The pivoting handle assemblies 130 are shown in the stowed position in Figure 1. The pivoting handle assemblies 130 are coupled to the respective rear uprights 114 of the rear support assembly 108, and are movable relative to the rear uprights 114 between the stowed position and the engagement position. Like the front handle assemblies 128, the pivoting handle assemblies 130 are generally arranged for engagement by a caregiver during patient transport, and may advantageously be utilized in the engagement position when the patient transport apparatus 100 operates in the chair configuration to transport the patient across floor surfaces. In some versions, the pivoting handle assemblies 130 could be configured in a manner similar to that disclosed in U.S. Patent No. 6,648,343, previously referenced. Other configurations are contemplated.

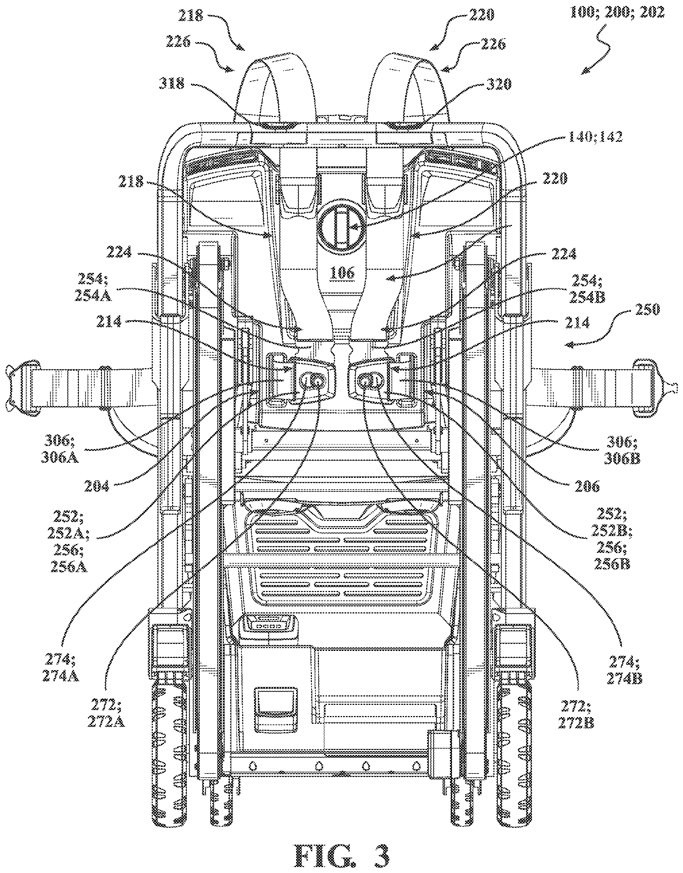

The handle assembly 132 is configured to be operated in a collapsed position and an extended position (not shown). The handle assembly 132 is shown in the collapsed position in Figure 1. The handle assembly 132 is also coupled to the rear support assembly 108, and generally comprises an upper grip 136 operatively attached to extension posts 138 which are supported within the respective rear uprights 114 for movement between the collapsed position and the extended position 132B. To this end, the extension posts 138 of the handle assembly 132 may be slidably supported by bushings, bearings, and the like coupled to the rear uprights 114, and may be lockable in and/or between the collapsed position and the extended position via an extension lock mechanism 140 with an extension lock release 142 arranged for engagement by the caregiver. As is best shown in Figure 3, the extension lock release 142 may be realized as a rotatable knob in communication with a flexible cable (not shown) which extends generally laterally between the rear uprights 114 and supports a cable connected to extension lock mechanisms 140 (not shown in detail) which releasably engage the extension posts 138 to maintain the handle assembly 132 between the extended position and the collapsed position. Here, it will be appreciated that the extension lock mechanism 140 and/or the extension lock release 142 could be of a number of different styles, types, configurations, and the like sufficient to facilitate selectively locking the handle assembly 132 in the extended position. In some versions, the handle assembly 132, the extension lock mechanism 140, and/or the extension lock release 142 could be configured in a manner similar to that disclosed in U.S. Patent No. 6,648,343, previously referenced. Other configurations are contemplated.

The illustrated patient transport apparatus 100 includes elements for transitioning between the chair configuration and the stair configuration. For example, in the representative version illustrated herein, the patient transport apparatus 100 includes a carrier assembly 148 arranged for movement relative to the support structure 102 between the chair configuration and the stair configuration. The carrier assembly 148 generally comprises at least one shaft 150 defining a wheel axis WA, one or more rear wheels 152 supported for rotation about the wheel axis WA, at least one track assembly 154 having a belt 156 for engaging stairs ST, and one or more hubs 158 supporting the shaft 150 and the track assembly 154 for concurrent pivoting movement about a hub axis HA. Here, movement of the carrier assembly 148 from the chair configuration to the stair configuration simultaneously deploys the track assembly 154 for engaging stairs with the belt 156 and moves the wheel axis WA longitudinally closer to the front support assembly 110 so as to position the rear wheels 152 further underneath the seat section 104 and closer to the front wheels 122. Operation along stairs ST is disclosed in U.S. Patent Application Publication No. 2021/0196535, previously referenced.

The illustrated patient transport apparatus 100 includes elements for aiding the transport of a patient across a floor surface and up and down stairs. For example, in the representative version illustrated herein, the track assemblies 154 each comprise a rail 168, the belt 156 of the track assemblies 154 being arranged for movement relative to the rail 168 to facilitate movement of the patient transport apparatus 100 up and down stairs. The patient transport apparatus 100 also comprises a drive system, generally indicated at 182, configured to facilitate driving the belts 156 of the track assemblies 154 relative to the rails 168 to facilitate movement of the patient transport apparatus 100 up and down stairs. The patient transport apparatus 100 comprises a control system and a user interface (not shown in detail) to, among other things, facilitate control of the track assemblies 154.

Referring to Figure 1, a patient containment system 200 to secure the patient to the patient transport apparatus 100 is generally shown. The patient containment system 200 may be removably engaged, coupled, or otherwise attached to the patient transport apparatus 100 to define a patient transport system 202 according to the present disclosure. Figures 1-4 depict the patient transport system 202 with the patient containment system 200 engaged with the patient transport apparatus 100. The patient containment system 200 may function to retain a patient supported for transport on the patient transport apparatus 100, particularly during transport along a floor surface, during ingress into or egress out of a structure (e.g., a home or building), and/or during transport up or down stairs.

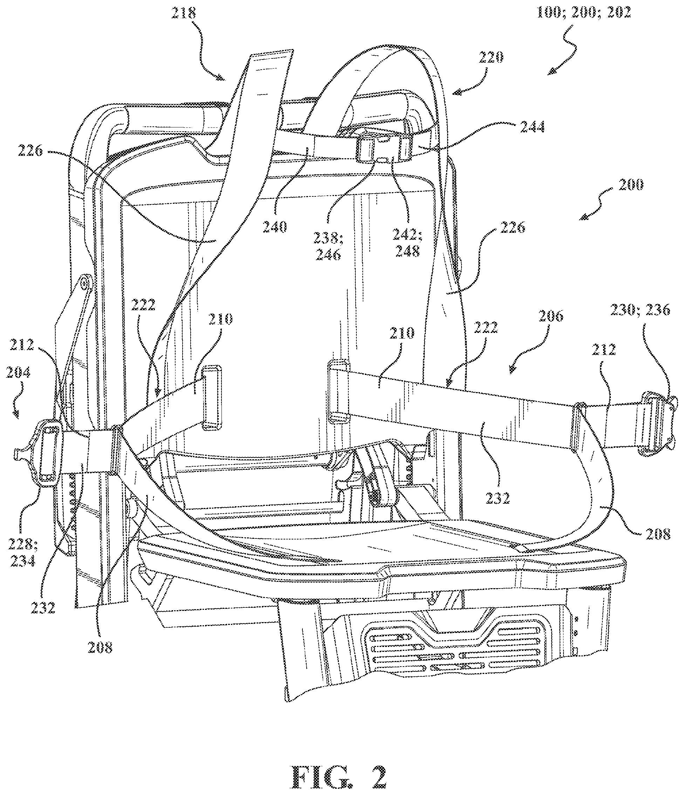

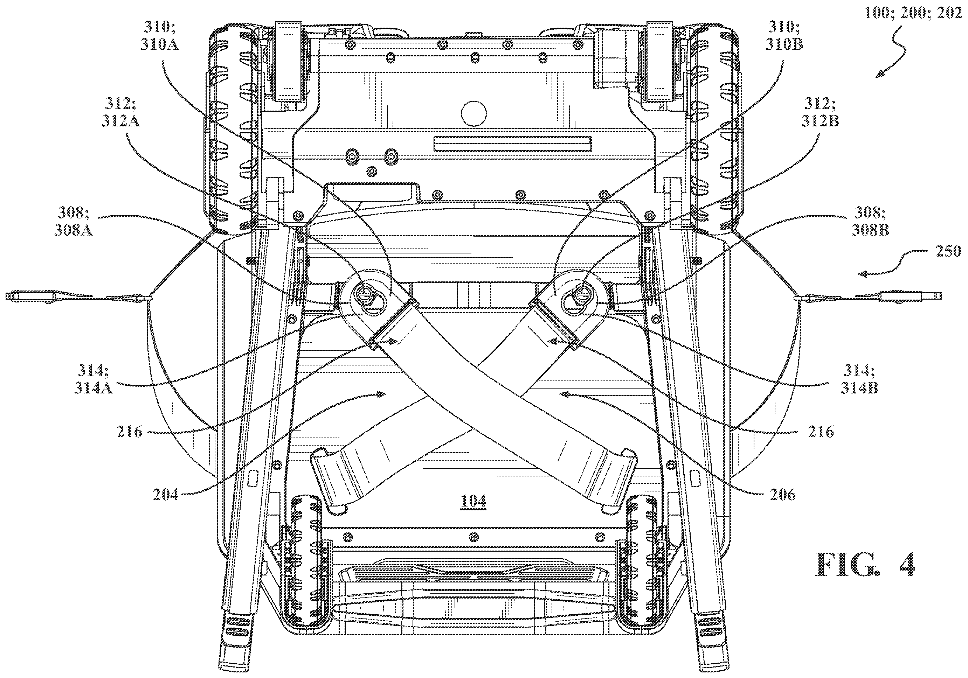

The patient containment system 200 is designed to secure patients of various sizes through the use of straps which are removably attachable to the patient transport apparatus 100. To this end, and as is best depicted in Figure 2, in some versions, the patient containment system 200 may comprise a first lower strap 204 and a second lower strap 206, each having a respective thigh region 208, waist region 210, and connection region 212 arranged between a back end 214 (shown in Figure 3) and a seat end 216 (shown in Figure 4). The connection region 212 is arranged between the thigh region 208 and the waist region 210, the thigh region 208 is arranged between the connection region 212 and the seat end 216, and the waist region 210 is arranged between the connection region 212 and the back end 214.

It will be appreciated that the thigh regions 208, the waist regions 210, and the connection regions 212 could be formed or otherwise defined in a number of different ways. By way of non-limiting example, the first and second lower straps 204, 206 could each be formed as unitary, one piece components between the back end 214 and the seat end 216, which taper or otherwise transition between the thigh region 208, the connection region 212, and the waist region 210. It is also contemplated that the first and second lower straps 204, 206 could be respectively formed from multiple components that are secured together (e.g., welded, bonded, adhered, and the like) and cooperate to define the thigh region 208, the connection region 212, and the waist region 210. By way of non-limiting example, a strap defining the connection width 348 could extend between the back end 214 and the seat end 216, and separate components that are secured to the strap could define the thigh region 208 and/or the waist region 210 (not shown). Other configurations are contemplated. In some versions one or more portions of the patient containment system 200 may comprise polymeric material, such as polyurethane, or other suitable materials to ease cleaning, and may comprise multiple materials (e.g., coated fabric). The components of the patient containment system 200 may provide a smooth, continuous, outer surface for wiping and thereby cleaning, and may be waterproof, water-resistant, and/or impervious to contaminants, such as dirt, grease, and body fluids. Other configurations are contemplated.

Referring to Figure 2, in some versions, the patient containment system 200 may comprise a first upper strap 218 and a second upper strap 220 each having a respective front end 222, back end 224 (see Figure 3), and shoulder region 226 extending between the front end 222 and the back end 224. As shown in Figure 1, the front end 222 of the first upper strap 218 and the front end 222 of the second upper strap 220 are coupled to the respective lower strap 204, 206.

Referring to Figure 2, a first connector 228 is coupled to the connection region 212 of the first lower strap 204, and a second connector 230 is coupled to the connection region 212 of the second lower strap 206. The first and second connectors 228, 230 may be coupled to the connection regions 212 via a guide 232, the guide 232 being configured to slide between the seat end 216 and the back end 214. The connectors 228, 230 cooperate to facilitate releasably attaching the first lower strap 204 and second lower strap 206 together to at least partially limit movement of the first lower strap 204 relative to the second lower strap 206 (and/or relative to each other). In the representative version illustrated herein, one of the first and second connectors 228, 230 is realized as a lower clasp 234, and the other of the first and second connectors 228, 230 is realized as a lower buckle 236 configured to releasably secure to the lower clasp 234. However, it will be appreciated that the first and second connectors 228, 230 could be configured in a number of different ways sufficient to limit movement between the first and second lower straps 204, 206.

Also shown in Figure 2, in some versions, a first shoulder connector 238 is coupled to the shoulder region 226 of the first upper strap 218 via an extension strap 240, and a second shoulder connector 242 is coupled to the shoulder region 226 of the second upper strap 220 via another extension strap 244. The second shoulder connector 242 is configured to releasably attach to the first shoulder connector 238 to at least partially limit movement of the first upper strap 218 relative to the second upper strap 220 (and/or relative to each other). In the representative version illustrated herein, one of the first and second shoulder connectors 238, 242 is realized as an upper clasp 246, and the other of the first and second shoulder connectors 238, 242 is realized as an upper buckle 248 configured to releasably secure to the upper clasp 246. However, it will be appreciated that the first and second shoulder connectors 238, 242 could be configured in a number of different ways sufficient to limit movement between the first and second upper straps 218, 220.

The second connector 228 is shaped to releasably attach to the first connector 230 and not to the first shoulder connector 238. Furthermore, the second shoulder connector 242 is shaped to releasably attach to the first shoulder connector 238 and not to the first connector 230. For example, in the representative version illustrated herein, the lower clasp 234 and the lower buckle 236 are of a first connector configuration, and the upper clasp 246 and the upper buckle 248 are of a second connector configuration that is different from the first connector configuration. It will be appreciated that the first connector configuration being different from the second connector configuration helps to prevent the first shoulder connector 238 or the second shoulder connector 242 from being attached to the first connector 228 or the second connector 230. This arrangement further promotes improved strap management and usability of the patient containment system 200 by preventing incorrect attachment. Similarly, in some versions, one or more components of the patient containment system 200 may include an identification indicia. The identification indicia may be designated colors, symbols, numbers, letters, tactile patterns, or a combination thereof. The identification indicia may be any identifying visual element or tactile surface which allows a user to easily identify each of the straps 204, 206, 218, 220 from one another.

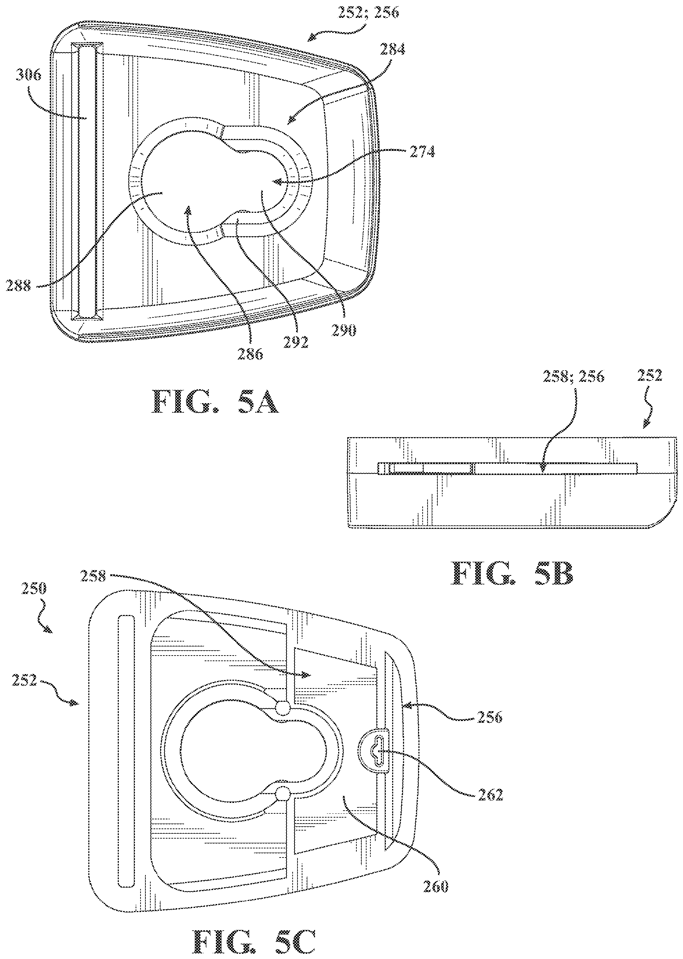

Referring now to Figures 3 and 4, in the representative version illustrated herein, the patient transport system 202 comprises a coupling system 250 to facilitate releasable attachment of portions of the patient containment system 200 to portions of the patient transport apparatus 100. Referring to Figure 3, the coupling system 250 includes a coupling bracket 252, a latch 254, and a keeper 256 (best shown in Figure 5C). The coupling bracket 252 is configured for removable attachment to the back section 106 of the patient transport apparatus 100. The latch 254 is coupled to the back end 224 of an upper strap 218, 220. The keeper 256 is operatively attached to the coupling bracket 252 and configured to removably receive the latch 254 to retain the upper strap 218, 220 coupled to the latch 254 together with the latch 254 relative to the coupling bracket 252. In the representative version shown in Figure 2, the coupling system 250 includes first coupling bracket 252A and a second coupling bracket 252B, a first latch 254A and a second latch 254B, and a first keeper 256A and a second keeper 256B. It should be noted that a shape and/or structure of the coupling bracket 252, the latch 254, and the keeper 256 and any other components of the coupling system 250 may vary and may be configured in other ways.

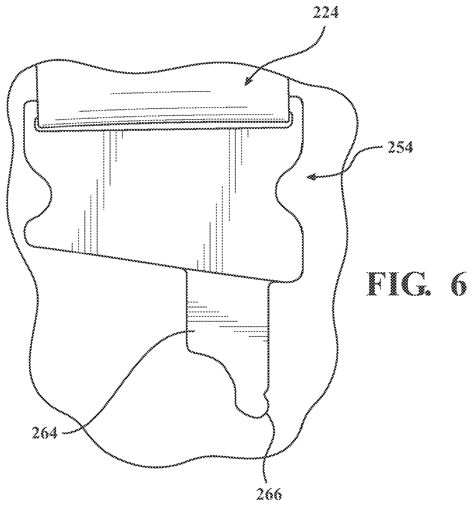

The coupling bracket 252, the keeper 256, and the latch 254 are further shown in Figures 5A-6. As shown in Figure 5B, the coupling bracket 252 defines an interior 258. Referring to Figure 5C, the keeper 256 is operatively attached to the coupling bracket 252 and defines a slot 260 formed extending into the interior 258. Also shown in Figure 5C, the slot 260 of the keeper 256 is shaped to receive at least a portion of the latch 254 therein and defines an abutment surface 262. Referring now to Figure 6, the latch includes a track 264 defining a retention face 266. It should be noted that, while the as coupling bracket 252 is depicted as a unitary, once-piece component throughout the drawings for illustrative purposes, other configurations are contemplated.

The coupling system 250 is configured to secure the upper straps 218, 220 to the patient transport apparatus 100. To accomplish this, the coupling system 250 secures the upper straps 218, 220 to the coupling bracket 252 and secures the coupling bracket 252 to the patient transport apparatus 100. In this way, the coupling system 250 is able to manage upper straps 218, 220 comprising an excess length as the upper straps 218, 220 are secured to the coupling bracket 252 and, therefore, the patient transport apparatus 100.

Removable reception of the latch 254 by the keeper 256 secures an upper strap 218, 220 (that is coupled to the latch 254) to the coupling bracket 252. As shown in Figure 3, the latch 254 is coupled to a back end 224 of an upper strap 218, 220. As previously stated, the keeper 256 is operatively attached to the coupling bracket 252 and is configured to removably receive the latch 254. As such, the keeper 256 is configured to receive the latch 254 and retain the latch 254 relative to the coupling bracket 252. Furthermore, the keeper 256, by retaining the latch 254 relative to the coupling bracket 252, retains the upper strap 218, 220 (that is coupled to the latch 254) together with the latch 254 relative to the coupling bracket 252.

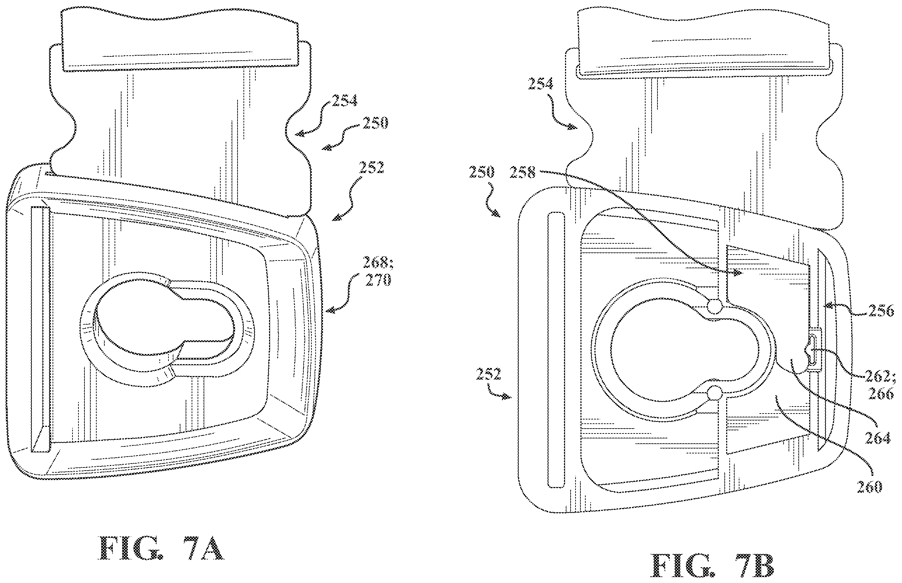

Removable reception of the latch 254 by the keeper 256 is shown in Figure 7A. To further illustrate the connection between the keeper 256 and the latch 254, Figure 7B depicts the interior 258 of the coupling bracket 252. As shown, the slot 260 of the keeper 256 is shaped to receive the latch 254 and receives, specifically, the track 264 of the keeper 256. Once the slot 260 receives the keeper 256, the retention face 266 contacts the abutment surface 262 to partially limit movement of the latch 254 relative to the keeper 256. In this way, the keeper 256 retains the latch 254 and the upper strap 218, 220 (that is coupled to the latch 254) relative to the coupling bracket 252.

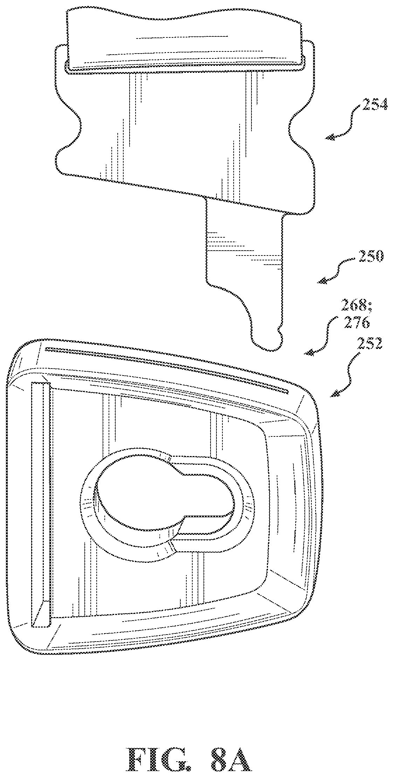

The coupling system 250 may further include an upper strap lock 268 interposed between the keeper 256 and the latch 254. In other words, the relationship between the latch 254 by the keeper 256 may be further defined as an upper strap lock 268. The upper strap lock 268 is selectively operable in a retained configuration 270 to at least partially limit movement of the latch 254 relative to the keeper 256. The retained configuration 270 is shown in Figure 8A, wherein the latch 254 is removably attached to the keeper 256 and the upper strap lock 268, limits movement of the latch 254 relative to the keeper 256 such that the latch 254 may be retained relative to the coupling bracket 252. It will be appreciated that the upper strap lock 268 could be configured to permit a limited amount of movement, or may be configured to substantially inhibit movement of the latch 254 relative to the keeper 256. While the latch 254 is removably attached to the keeper 256 in the retained configuration 270, in some instances, other components of the coupling system 250 may also be attached to the coupling bracket 252 in the retained configuration 270. For example, an anchor 272 that is operatively attached to the patient transport apparatus 100 may be received by a receiver 274 of the coupling bracket 252 in the retained configuration 270, as described in greater detail below.

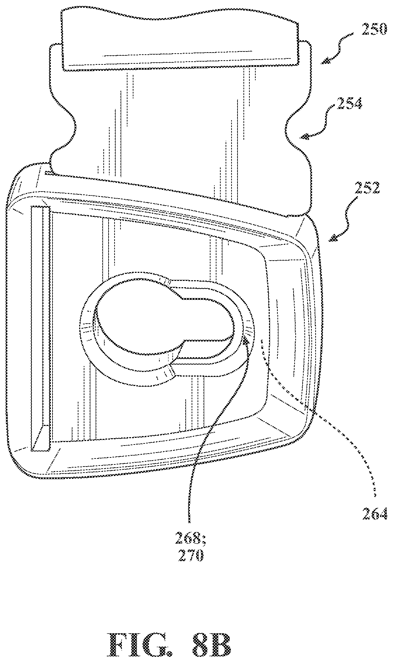

The upper strap lock 268 is also selectively operable in a released configuration 276 to permit movement of the latch 254 relative to the keeper. The released configuration 276 is shown in Figure 8B, wherein the latch 254 is not attached to the keeper 256, and the upper strap lock 268 permits movement of the latch 254 relative to the keeper 256. While the latch 254 is not attached to the keeper 256 in the released configuration 276, in some instances, other components of the coupling system 250 may be attached to the coupling bracket 252 in the released configuration 276. For example, an anchor 272 that is operatively attached to the patient transport apparatus 100 may be received by a receiver 274 of the coupling bracket 252 in the released configuration 276, as described in greater detail below.

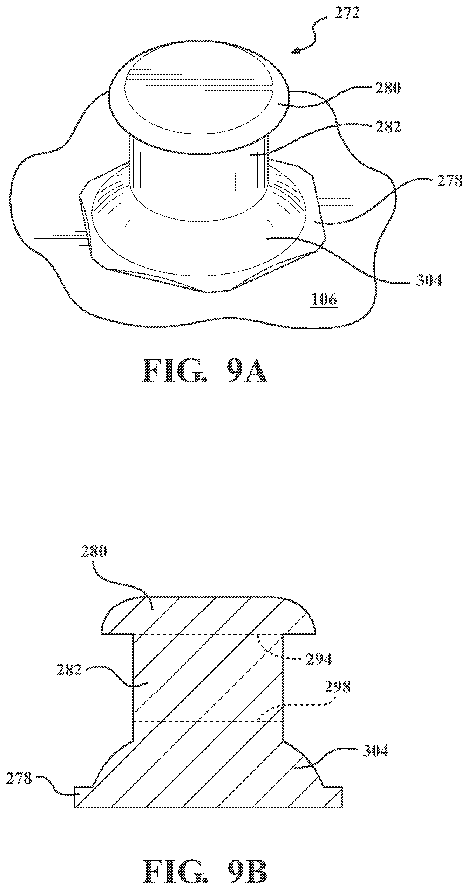

As noted above, the coupling bracket 252 may be removably attached to the patient transport apparatus 100. The coupling system 250 may include an anchor 272 (shown in Figure 9A) and a receiver 274 (shown in Figure 5A) to facilitate the removable attachment of the coupling bracket 252 to the back section 106 of the patient transport apparatus 100. In the representative version shown in Figure 2, the coupling system 250 includes a first anchor 272A, a second anchor 272B, a first receiver 274A, and a second receiver 274B. It should be noted that the patient transport apparatus 100 may include any suitable number of anchors, receivers, and the like.

As shown in Figure 9A, the anchor 272 is operatively attached to the patient transport apparatus 100 and, as shown in Figure 5A, the receiver 274 is operatively attached to the coupling bracket 252. However, it will be appreciated that this arrangement could be interchanged in part or in full. For example, in other instances (not shown), one or more anchors 272 may be operatively attached to the coupling bracket 252. Similarly, one or more receivers 274 may be coupled to the patient transport apparatus 100.

Additional components of the anchor 272 are shown in Figure 9A. Here, the anchor 272 may comprise an anchor base 278, an anchor head 280, and an anchor shaft 282 extending between the anchor base 278 and the anchor head 280. In the representative version illustrated herein, the anchor 272 is operatively attached to the patient transport apparatus 100. It should be noted that the anchor 272 may include any additional components suitable for attaching the anchor 272 to the patient transport apparatus 100. In other instances, the anchor 272 may be operatively attached to the coupling bracket 252. In such instances, the anchor 272 may include any other suitable components for operatively attaching to the coupling bracket 252. Here, too, the anchor 272 could be defined by a part of a rivet. While the anchors 272 are depicted as unitary, once-piece components throughout the drawings for illustrative purposes, and other configurations are contemplated.

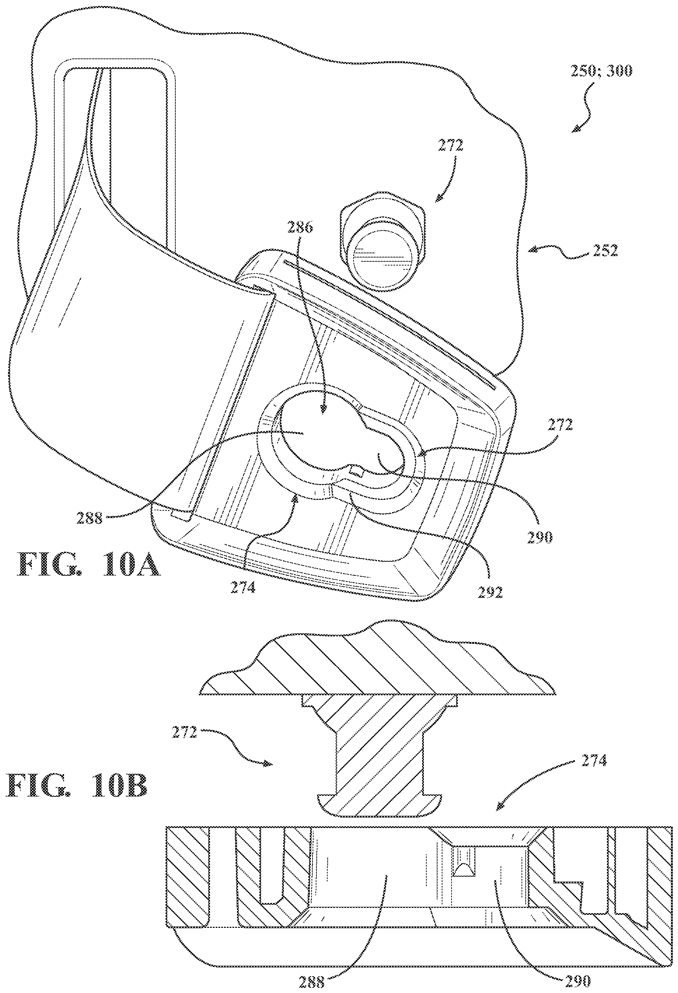

Additional components of the receiver 274 are shown in Figure 5A. Here, the receiver 274 comprises a receiver body 284 defining a receiver aperture 286. The receiver aperture 286 has a first aperture region 288 shaped to receive the anchor head 280 of the anchor 272 therethrough, and a second aperture region 290 shaped to receive the anchor shaft 282 therein. In some versions, the receiver 274 may comprise a tab 292 formed extending into the receiver aperture 286 and arranged adjacent to the second aperture region 290 to at least partially restrict movement of the anchor shaft 282 between the second aperture region 290 and the first aperture region 288. Put differently, the tab 292 helps retain the coupling system 250 in an engaged configuration 296 (see Figure 10B) by creating resistance to movement of the anchor shaft 282 from the second aperture region 290 to the first aperture region 288.

As is best depicted schematically in Figure 9B, the anchor head 280 is sized radially larger than the anchor shaft 282. In some versions, the anchor head 280 may define a head perimeter 294, and the anchor shaft 282 may define a shaft perimeter 298 that is smaller than the head perimeter 294. Here, the first aperture region 288 and the second aperture region 290 are sized relative to the head perimeter 294 such that the anchor head 280 can pass through the first aperture region 288 but cannot pass through the second aperture region 290. In the illustrated versions, the anchor 272 is also provided with a tapered region 304 which is disposed between the anchor base 278 and the anchor shaft 282, and the receiver 274 includes tapered profiles leading into the receiver apertures 286 to abut the tapered regions 304. This arrangement allows for bearing contact which can be used to facilitate adjusting the first and second lower straps 204, 206 between different orientations, angles, and the like.

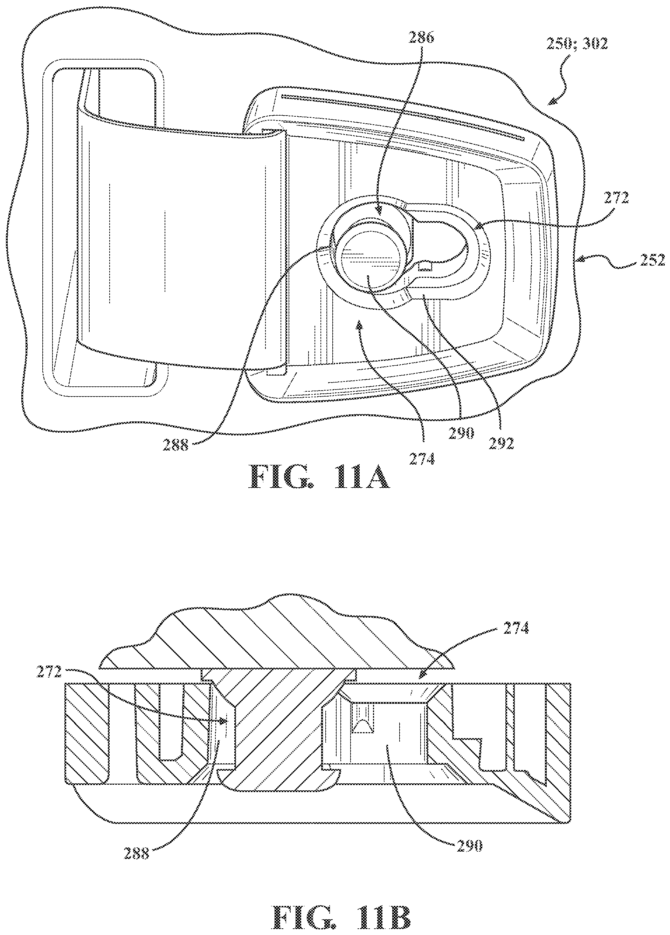

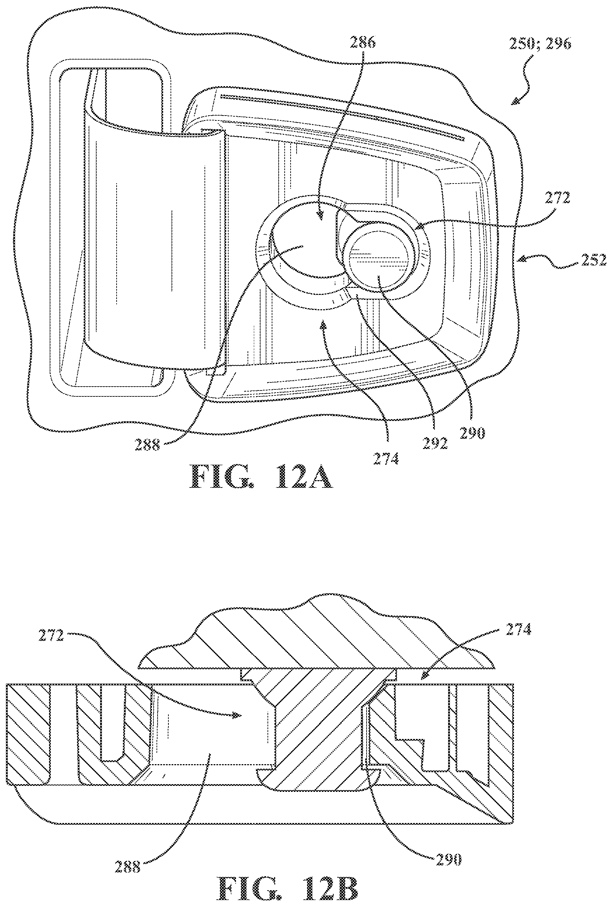

The coupling system 250 is operable between an engaged configuration 296 to at least partially limit movement of the anchor 272 relative to the receiver 274 (see Figures 12A and 12B), a disengaged configuration 300 to release the anchor 272 from the receiver 274 (see Figures 10A and 10B), and a transitional configuration 302 (see Figures 11A and 11B) to facilitate movement between the engaged configuration 296 and the disengaged configuration 300. It should be noted that, in the instances of Figures 10A-12B, the latch 254 is not illustrated as being attached to the keeper 256 in the engaged configuration 296, the disengaged configuration 300, and the transitional configuration 302. However, in some instances, the latch 254 may be attached to the keeper 256 in one or more of these configurations.

In some instances, the coupling system 250 may be configured to secure the lower straps 204, 206 and the upper straps 218, 220 to one another, and to secure the straps 204, 206, 218, 220 to the patient transport apparatus 100. As noted above, the coupling system 250 secures the upper straps 218, 220 to the coupling bracket 252, and also secures the coupling bracket 252 to the patient transport apparatus 100. As such, in instances where the coupling bracket 252 is coupled to the lower straps 204, 206, the lower straps 204, 206 and the upper straps 218, 220 are secured to one another when the coupling system 250 secures the upper straps 218, 220 to the coupling bracket 252. Furthermore, when the coupling system 250 secures the coupling bracket 252 to the patient transport apparatus 100, the straps 204, 206, 218, 220 are secured to the patient transport apparatus 100. As will be described in this section, the coupling system 250 may also include additional components to facilitate securing the straps 204, 206, 218, 220 to the patient transport apparatus 100.

Figure 3 illustrates an instance where the coupling bracket 252 is coupled to the lower straps 204, 206. As shown, the coupling bracket 252 may further include a strap mount 306 configured to receive the back end 214 of a respective lower strap 204, 206. In the representative version shown in Figure 3, each coupling bracket 252 may include a back section strap mount 306 (best shown in Figure 5A) such that the coupling system 250 includes a first back section strap mount 306A and a second back section strap mount 306B. In instances where the coupling bracket 252 is coupled to the back end 214 of a respective lower strap 204, 206, the coupling bracket 252 may be further defined as a back section coupling bracket 252. Here, the anchor 272 may be further defined as a back section anchor 272 operatively attached to the back section 106 of the patient transport apparatus 100 and the receiver 274 may be further defined as a back section receiver 274 operatively attached to the back section coupling bracket 252.

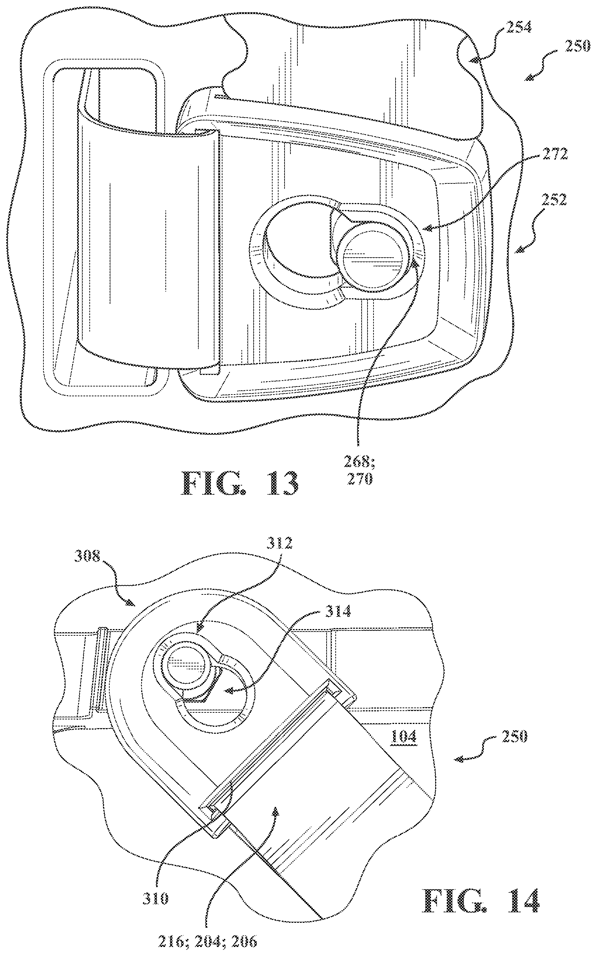

Referring to Figure 4, the coupling system 250 may include a seat section coupling bracket 308 to facilitate securing a respective lower strap 204, 206 to the patient transport apparatus 100. Referring to Figure 14, the seat section coupling bracket 308 may include a seat section strap mount 310 configured to receive the seat end 216 of a respective lower strap 204, 206. Additionally, the coupling system 250 may also include a seat section anchor 312 operatively attached to the seat section 104 of the patient transport apparatus 100 and a seat section receiver 314 operatively attached to the seat section coupling bracket 308 and shaped to releasably secure the seat section anchor 312. In the representative version shown in Figure 4, the coupling system 250 includes a first seat section coupling bracket 308A and a second seat section coupling bracket 308B, a first back section strap mount 310A and a second back section strap mount 310B, a first seat section anchor 312A and a second seat section anchor 312B, and a first seat section receiver 314A and a second seat section receiver 314B.

The seat section coupling bracket 308 operates in a similar fashion to the back section coupling bracket 252. Specifically, just as the back section receiver 274 of the back section coupling bracket 252 is configured to receive the back section anchor 272, the seat section receiver 314 of the seat section coupling bracket 308 is configured to receive the seat section anchor 312. The seat section coupling bracket 308 and the seat section receiver 314, and the seat section anchor 312 may include any suitable shape and structure to facilitate reception of the seat section anchor 312 by the seat section receiver 314. Furthermore, a shape and structure of the seat section coupling bracket 308, the seat section receiver 314, and the seat section anchor 312 may vary from a shape and structure of the back section coupling bracket 252, the back section receiver 274, and the back section anchor 272.

As previously stated, the coupling system 250 is operable between an engaged configuration 296 to at least partially limit movement of the anchor 272 relative to the receiver 274 (see Figures 12A and 12B), a disengaged configuration 300 to release the anchor 272 from the receiver 274 (see Figures 10A and 10B), and a transitional configuration 302 (see Figures 11A and 11B) to facilitate movement between the engaged configuration 296 and the disengaged configuration 300. Additionally, the upper strap lock 268 is selectively operable between a retained configuration 270 (shown in Figure 8B) to at least partially limit movement of the latch 254 relative to the keeper 256 and a released configuration 276 (shown in Figure 8A) to permit movement of the latch 254 relative to the keeper 256.

**In** some instances, the patient containment system 200 may be configured to inhibit the coupling system 250 from moving from one operating state to another. For example, while the coupling system 250 is operating in the engaged configuration 296 and the upper strap lock 268 is operating in the retained configuration 270, the coupling system 250 may be inhibited from moving from the engaged configuration 296 to the disengaged configuration 300. In this way, the patient containment system 200 prevents the coupling bracket 252 from being removed from the patient transport apparatus 100 while the latch 254 is received by the keeper 256. As such, a user of the patient containment system 200 first removes the latch 254 from the anchor 272, moving the upper strap lock 268 from the retained configuration 270 to the released configuration 276, prior to removing the coupling bracket 252 from the patient transport apparatus 100 and moving the coupling system 250 from the engaged configuration 296 to the disengaged configuration 300.

It should be noted that, in instances where either the coupling system 250 is operating in the disengaged configuration 300 and/or the upper strap lock 268 is operating in the released configuration 276, neither the coupling system 250 nor the upper strap lock 268 are inhibited from moving to a different configuration. In other words, the coupling bracket 252 may be attached to the patient transport apparatus 100 and the keeper 256 may receive the latch 254 in any suitable order.

In some configurations, a shape of the latch 254, a shape of the keeper 256, a shape of the coupling bracket 252, a shape of the receiver 274, and/or a shape of the anchor 272 may provide an order for attaching the coupling bracket 252 to the patient transport apparatus 100 and for the receiver 274 receiving the latch 254. For instance, in some instances, a shape of the anchor 272 and a shape of the latch 254 may inhibit the upper strap lock 268 from moving from the released configuration 276 to the retained configuration 270 during operation of the coupling system 250 in the engaged configuration 296. As an example, a shape of the anchor 272 and a shape of the latch 254 may cause the latch 254 to abut the anchor 272 while the coupling system 250 is operating in the engaged configuration 296, inhibiting the keeper 256 from receiving the latch 254. In such an instance, the keeper 256 receives the latch 254, moving the upper strap lock 268 from the released configuration 276 to the retained configuration 270, prior to the coupling bracket 252 being attached to the patient transport apparatus 100 and moving the coupling system 250 from the disengaged configuration 300 to the engaged configuration 296.

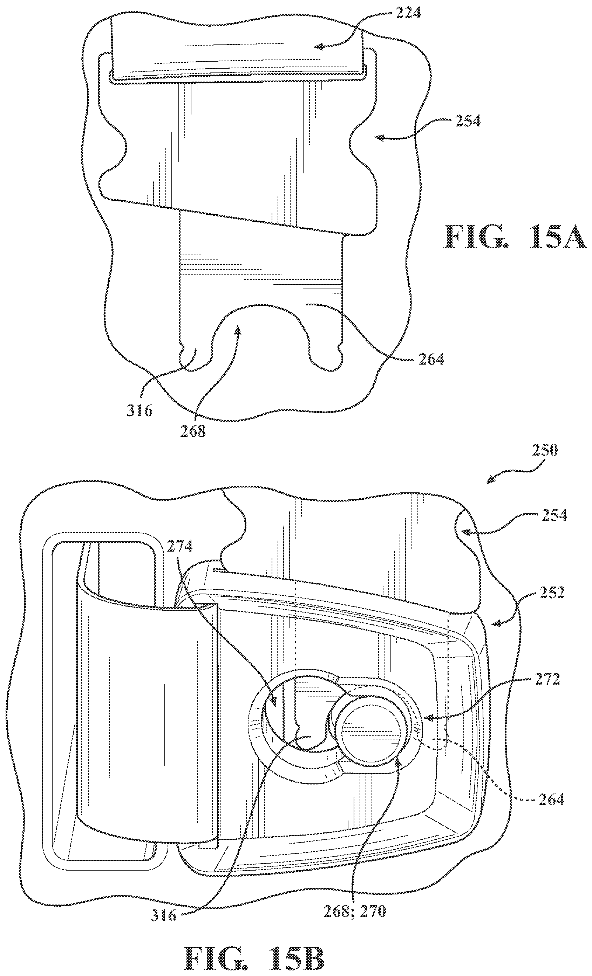

In some versions, the upper strap lock 268 may include a blocking tab 316 to facilitate inhibiting the coupling system 250 from moving from the engaged configuration 296 to the disengaged configuration 300 during operation of the upper strap lock 268 in the retained configuration 270. Figure 15A illustrates an instance where the upper strap lock 268 includes the blocking tab 316. In the instance of Figure 15A, the blocking tab 316 is operatively attached to the track 264 of the latch 254. However, in other instances, the blocking tab 316 may be a separate component or may be attached to any suitable component of the patient containment system 200.

Figure 15B illustrates operation of the blocking tab 316. As shown, in the retained configuration 270, the blocking tab 316 extends into the receiver 274. Furthermore, the blocking tab 316 is adjacent to the anchor 272 in the retained configuration 270 and inhibits the coupling system 250 from moving from the engaged configuration 296 to the disengaged configuration 300. In the version depicted in Figures 15A and 15B, the blocking tab 316 inhibits the coupling system 250 from moving from the disengaged configuration 300 to the engaged configuration 296 during operating of the upper strap lock 268 in the retained configuration 270. Here, during operation of the upper strap lock 268 in the retained configuration 270, the blocking tab 316 extends into the receiver 274, preventing the coupling bracket 252 from being attached to the anchor 272 and the patient transport apparatus 100.

Referring to Figure 3, in the representative version illustrated herein, the patient transport apparatus 100 includes first and second webbing adjusters 318, 320 to facilitate securing the upper straps 218, 220. The first and second webbing adjusters 318, 320 may be operatively attached to the back section 106 and shaped to receive the back ends 224 of the first and second upper straps 218, 220, respectively, therethrough. Here, the first and second webbing adjusters 318, 320 are configured to releasably engage the shoulder regions 226 of the first and second upper straps 218, 220, respectively, to adjust tension in the first and second upper straps 218, 220 (e.g., between the first and second webbing adjusters 318, 320 and the respective front ends 222). It will be appreciated that the first and second webbing adjusters 318, 320 could be configured in a number of different ways sufficient to engage the shoulder regions 226 of the first and second upper straps 218, 220.

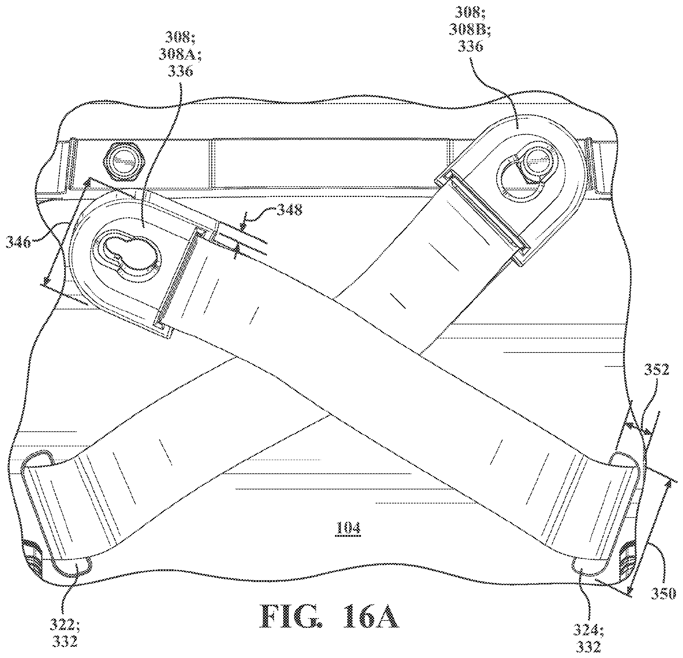

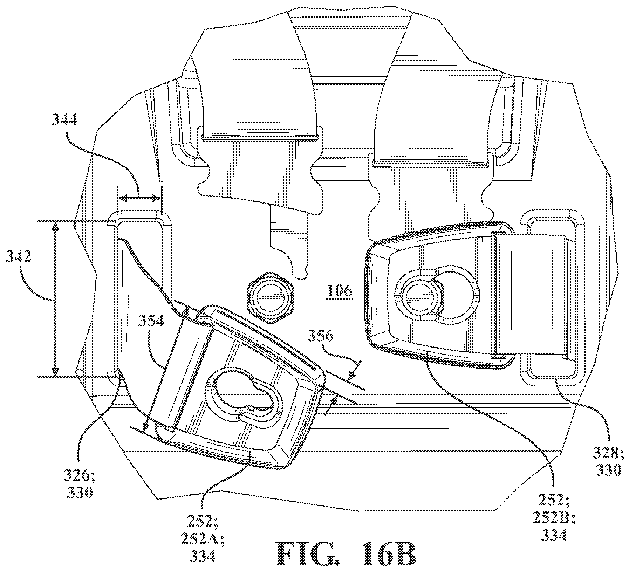

Referring to Figure 16A, the seat section 104 of patient transport apparatus 100 may define a first seat section aperture 322 and a second seat section aperture 324 shaped to receive the seat section coupling bracket 308 therethrough. As shown in Figure 16B, the back section 106 of the patient transport apparatus 100 defines a first back section aperture 326 and a second back section aperture 328 shaped to receive the back section coupling bracket therethrough. By receiving the seat section coupling bracket 308 and the back section coupling bracket 252 therethrough, the seat section apertures 322, 324 and the back section apertures 326, 328, respectively, facilitate securing the seat section coupling bracket 308 and the back section coupling bracket 252 to the patient transport apparatus 100. The seat section apertures 322, 324 and the back section apertures 326, 328 may be shaped to ensure proper use of the patient containment system 200. For example, the seat section apertures 322, 324 may be shaped to prevent receiving the back section coupling bracket 252 therethrough. Similarly, the back section apertures 326, 328 may be shaped to prevent receiving the seat section coupling bracket 308 therethrough.

In some versions, the seat section apertures 322, 324 may define a seat section aperture profile 332 shaped to allow the seat section coupling bracket 308 to pass through and to prevent the back section coupling bracket 252 from passing through. Similarly, the back section apertures 326, 328 may define a back section aperture profile 330 shaped to allow the back section coupling bracket 252 to pass through and to prevent the seat section coupling bracket 308 from passing through. In some versions, the back section coupling bracket 252 may define a back section coupling bracket profile 334 and the seat section coupling bracket 308 may define a seat section coupling bracket profile 336. Here, the seat section apertures 322, 324 and the back section apertures 326, 328 may be shaped such that a cross-sectional area of the back section coupling bracket profile 334 is larger in at least one dimension than a cross-sectional area of seat section aperture 332 and a cross-sectional area of the seat section coupling bracket profile 336 is larger in at least one dimension than a cross-sectional area of the back section aperture profile 330, as described in greater detail below. The back section coupling bracket profile 334 may include a back section coupling bracket length 338 and a back section coupling bracket width 340, the back section aperture profile 330 may include a back section aperture length 342 and a back section aperture width 344, the seat section coupling bracket profile 336 may include a seat section coupling bracket length 346 and a seat section coupling bracket width 348, and the seat section aperture profile 332 may include a seat section aperture length 350 and a seat section aperture width 352.

It will be appreciated that the dimensions of the aperture profiles 334, 330, 336, 332 may be sized to ensure that the seat section apertures 322, 324 are able to receive the seat section coupling bracket 308 therethrough and to ensure that the back section apertures 326, 328 are able to receive the back section coupling bracket 252 therethrough. For example, in the instance of Figures 16A and 16B, the back section coupling bracket length 338 may be smaller than the back section aperture length 342, the back section coupling bracket width 356 may be smaller than the back section aperture width 344, the seat section coupling bracket length 346 may be smaller than the seat section aperture length 350, and the seat section coupling bracket width 348 may be smaller than the seat section aperture width 352. However, it will be appreciated that other configurations are contemplated. The dimensions of the aperture profiles 334, 330, 336, 332 may be selected to prevent the seat section apertures 322, 324 from receiving the back section coupling bracket 252 therethrough and prevent the back section apertures 326, 328 from receiving the seat section coupling bracket 308 therethrough. In some versions, the back section coupling bracket length 354 may be larger than the seat section coupling aperture length 350. In some versions, the back section coupling bracket width 356 may be larger than the seat section aperture width 352. In some versions, the seat section coupling bracket length 346 may be larger than the back section aperture length 342. In some versions, the seat section coupling bracket width 348 may be larger than the back section aperture width 344.

As shown in Figure 14, in the illustrated version, the patient transport apparatus 100 comprises first and second webbing adjusters 318, 320 operatively attached to the back section 106, disposed vertically above the first and second back apertures 326, 328, and shaped to receive the back ends 224 of the first and second upper straps 218, 220, respectively, therethrough. Here, the first and second webbing adjusters 318, 320 are configured to releasably engage the shoulder regions 226 of the first and second upper straps 218, 220, respectively, to adjust tension in the first and second upper straps 218, 220 (e.g., between the first and second webbing adjusters 318, 320 and the respective front ends 222). It will be appreciated that the first and second webbing adjusters 318, 320 could be configured in a number of different ways sufficient to engage the shoulder regions 226 of the first and second upper straps 218, 220.

**In** some versions, the first and second upper straps 218, 220 may comprise strap handles (not shown) coupled to the shoulder region 226 and arranged for engagement by the patient. Here, the patient can grasp the strap handles during transport. Furthermore, if the patient has a decreased level of consciousness, the caregiver may use the strap handles to locate and/or retain the patient's hands or wrists during transport, which advantageously helps position the patient ideally for transport in certain use cases where there is less than optimal room on the lateral sides of the patient transport apparatus 100 (e.g., when traveling down a narrow stairwell).

As is best shown in Figures 15A-15B, in some versions, a first shoulder connector 238 is coupled to the shoulder region 226 of the first upper strap 218 via an extension strap (not shown in detail), and a second shoulder connector 242 is coupled to the shoulder region 226 of the second upper strap 220 via another extension strap (not shown in detail). The second shoulder connector 242 is configured to releasably attach to the first shoulder connector 238 to at least partially limit movement of the first upper strap 218 relative to the second upper strap 220 (and/or relative to each other). In the representative version illustrated herein, one of the first and second shoulder connectors 238, 242 is realized as an upper clasp 246, and the other of the first and second shoulder connectors 238, 242 is realized as an upper buckle 248 configured to releasably secure to the upper clasp 246. However, it will be appreciated that the first and second shoulder connectors 238, 242 could be configured in a number of different ways sufficient to limit movement between the first and second upper straps 218, 220.

It will be appreciated that the patient containment system 200 may comprise additional straps, sections, and the like to facilitate securing a patient for transport on the patient transport apparatus 100. For example, additional straps may be provided to wrap around the patient's chest. Other configurations are contemplated. In some versions, a strap management box (not shown) may be coupled to the patient transport apparatus 100 to stow one or more components of the patient containment system 200 when not in use.

Several configurations have been discussed in the foregoing description. However, the configurations discussed herein are not intended to be exhaustive or limit the invention to any particular form. The terminology which has been used is intended to be in the nature of words of description rather than of limitation. Many modifications and variations are possible in light of the above teachings, and the invention may be practiced otherwise than as specifically described.

## Claims

1. A patient transport system (202) comprising:
a patient transport apparatus (100) comprising a seat section (104) and a back section (106) for supporting the patient; and
a patient containment system (200) including a lower strap (204, 206), an upper strap (218, 220) having a front end (222) coupled to the lower strap (204, 206) and a back end (224), and
a coupling system (250) to facilitate releasable attachment of the patient containment system (200) to the patient transport apparatus (100), the coupling system (250) including:
a latch (254) coupled to the back end (224) of the upper strap (218, 220),
a coupling bracket (252) configured for removable attachment to the back section (106) of the patient transport apparatus (100), and
a keeper (256) operatively attached to the coupling bracket (252) and configured to removably receive the latch (254) to retain the upper strap (218, 220) together with the latch (254) relative to the coupling bracket (252);
wherein the coupling bracket (252) defines an interior (258), with the keeper (256) defining a slot (260) formed extending into the interior (258) of the coupling bracket (252) and shaped to receive at least a portion of the latch (254) therein to retain the upper strap (218, 220) together with the latch (254) relative to the coupling bracket (252).

2. The patient transport system (202) of claim 1,
wherein the slot (260) of the keeper (256) defines an abutment surface (262); and
wherein the latch (254) includes a track (264) defining a retention face (266) arranged to contact the abutment surface (262) of the slot (260) to at least partially limit movement of the latch (254) relative to the keeper (256).

3. The patient transport system (202) of claim 2,
wherein the coupling system (250) further includes:
an anchor (272) operatively attached to one of the patient transport apparatus (100) and the coupling bracket (252); and
a receiver (274) operatively attached to the other of the patient transport apparatus (100) and the coupling bracket (252), the receiver (274) being configured to releasably secure the anchor (272) to facilitate removable attachment of the patient containment system (200) to the back section (106) of the patient transport apparatus (100).

4. The patient transport system (202) of claim 3,
wherein the coupling system (250) is operable between:
an engaged configuration (296) where the receiver (274) secures the anchor (272) to at least partially limit movement of the coupling bracket (252) relative the back section (106); and
a disengaged configuration (300) where the receiver (274) releases the anchor (272) to facilitate removing the coupling bracket (252) from the back section (106).

5. The patient transport system (202) of claim 4,
wherein the coupling system (250) further includes:
an upper strap lock (268) interposed between the keeper (256) and the latch (254) and being selectively operable between:
a retained configuration (270) to at least partially limit movement of the latch (254) relative to the keeper (256); and
a released configuration (276) to permit movement of the latch (254) relative to the keeper (256); optionally
wherein the coupling system (250) is inhibited from moving from the engaged configuration (296) to the disengaged configuration (300) during operation of the upper strap lock (268) in the retained configuration (270).

6. The patient transport system (202) of claim 5,
wherein the upper strap lock (268) includes a blocking tab (316) operatively attached to the track (264) of the latch (254); and optionally,
wherein the blocking tab (316) extends into the receiver (274) in the retained configuration (270); and optionally,
wherein the blocking tab (316) is adjacent to the anchor (272) in the retained configuration (270) and inhibits the coupling system (250) from moving from the engaged configuration (296) to the disengaged configuration (300).

7. The patient transport system (202) of any one of claims 3 - 6,
wherein the anchor (272) includes an anchor base (278), an anchor head (280), and an anchor shaft (282) extending between the anchor base (278) and the anchor head; and
wherein the receiver (274) includes a receiver body (284) defining a receiver aperture (286) having a first aperture region (288) shaped to receive the anchor head (280) therethrough, and having a second aperture region (290) disposed in communication with the first aperture region (288) and being shaped to receive the anchor shaft (282) therein; and optionally,
wherein the receiver (274) further includes a tab (292) formed extending into the receiver aperture (286) and arranged adjacent to the second aperture region (290) to at least partially restrict movement of the coupling bracket (252) between the first aperture region (288) and the second aperture region (290).

8. The patient transport system (202) of any one of claims 3 - 7,
wherein the lower strap (204, 206) includes a seat end (216) and a back end (224);
wherein the coupling bracket (252) is further defined as a back section coupling bracket (252) coupled to the back end (224) of the lower strap (204, 206);
wherein the anchor (272) is further defined as a back section anchor (272) operatively attached to the back section (106) of the patient transport apparatus (100);
wherein the receiver (274) is further defined as a back section receiver (274) operatively attached to the back section coupling bracket (252); and
wherein the coupling system (250) further includes:
a seat section anchor (312) operatively attached to the seat section (104) of the patient transport apparatus (100), and
a seat section receiver (314) shaped to releasably secure the seat section anchor (312).

9. The patient transport system (202) of claim 8,
wherein the back section coupling bracket (252) further includes a strap mount (306) configured to receive the back end (224) of the lower strap (204, 206).

10. The patient transport system (202) of any one of claims 8 - 9,
wherein the coupling system (250) includes a seat section coupling bracket (308) coupled to the seat end (216) of the lower strap (204, 206), with the seat section receiver (314) being operatively attached to the seat section coupling bracket (308).

11. The patient transport system (202) of claim 10,
wherein the back section (106) of the patient transport apparatus (100) further includes a back section aperture shaped to receive the back section coupling bracket (252) therethrough and to prevent receiving the seat section coupling bracket (308) therethrough; and
wherein the seat section (104) of the patient transport apparatus (100) further includes a seat section aperture (322, 324) shaped to receive the seat section coupling bracket (308) therethrough and to prevent receiving the back section coupling bracket (252) therethrough.

12. The patient transport system (202) of claim 11,
(i) wherein the back section aperture defines a back section aperture profile (330) shaped to allow the back section coupling bracket (252) to pass through the back section aperture and to prevent the seat section coupling bracket (308) from passing through the back section aperture; and
wherein the seat section aperture (322, 324) defines a seat section aperture profile (332) shaped to allow the seat section coupling bracket (308) to pass through the seat section aperture (322, 324) and to prevent the back section coupling bracket (252) from passing through the seat section aperture (322, 324); or
(ii) wherein the back section coupling bracket (252) defines a back section coupling bracket profile (334);
wherein the back section aperture defines a back section aperture profile (330);
wherein the seat section coupling bracket (308) defines a seat section coupling bracket profile (336);
wherein the seat section aperture (322, 324) defines a seat section aperture profile (332);
wherein a cross-sectional area of the back section coupling bracket profile (334) is larger in at least one dimension than a cross-sectional area of the seat section aperture profile (332); and
wherein a cross-sectional area of the seat section coupling bracket profile (336) is larger in at least one dimension than a cross-sectional area of the back section aperture profile (330).

13. The patient transport system (202) of claim 11 or 12,
wherein the back section coupling bracket (252) defines a back section coupling bracket profile (334) including a back section coupling bracket length (338) and a back section coupling bracket width (340);
wherein the back section aperture defines a back section aperture profile (330) including a back section aperture length (342) and a back section aperture width (344);
wherein the seat section coupling bracket (308) defines a seat section coupling bracket profile (336) including a seat section coupling bracket length (346) and a seat section coupling bracket width (348);
wherein the seat section aperture (322, 324) defines a seat section aperture profile (332) including a seat section aperture length (350) and a seat section aperture width (352);
wherein the back section coupling bracket length (338) is smaller than the back section aperture length (342) and the back section coupling bracket width (340) is smaller than the back section aperture width (344); and
wherein the seat section coupling bracket length is smaller than the seat section aperture length (350) and the seat section coupling bracket width is smaller than the seat section aperture width (352); and optionally,
wherein at least one of the following conditions is fulfilled:
(i) the back section coupling bracket length (338) is larger than the seat section aperture length (350);
(ii) the back section coupling bracket width (340) is larger than the seat section aperture width (352);
(iii) the seat section coupling bracket length is larger than the back section aperture length (342);
(iv) the seat section coupling bracket width is larger than the back section aperture width (344).

14. The patient transport system (202) of one of the preceding claims,
wherein the upper strap (218, 220) includes a shoulder region (226) extending between the front end (222) and the back end (224); and
wherein the patient transport apparatus (100) further comprises a webbing adjuster (318, 320) operatively attached to the back section (106) and shaped to receive the back end (224) of the upper strap (218, 220) therethrough, the webbing adjuster (318, 320) being configured to releasably engage the shoulder region (226) of the upper strap (218, 220) to adjust tension in the upper strap (218, 220) between the webbing adjuster (318, 320) and the lower strap (204, 206).

15. The patient transport system (202) of one of the preceding claims,
wherein the lower strap (204, 206) is further defined as a first lower strap (204), wherein the upper strap (218, 220) is further defined as a first upper strap (218), and wherein the patient containment system (200) further comprises a second lower strap (206) and a second upper strap (220), and optionally,
wherein at least one of the following conditions is fulfilled:
(i) each of the first lower strap (204) and the second lower strap (206) have a thigh region (208), a waist region (210), and a lower connection region arranged between the thigh region (208) and the waist region (210);
wherein each of the first upper strap (218) and the second upper strap (220) have an upper connection region (212) arranged between the front end (222) and the back end (224); and
wherein the patient containment system (200) further comprises:
a first connector (228) coupled to the lower connection region (212) of the first lower strap (204);
a second connector (230) coupled to the lower connection region (212) of the second lower strap (206) and configured to releasably attach to the first connector (228) to at least partially limit movement of the first lower strap (204) relative to the second lower strap (206);
a third connector coupled to the upper connection region (212) of the first upper strap (218); and
a fourth connector coupled to the upper connection region (212) of the second upper strap (220) and configured to releasably attach to the third connector to at least partially limit movement of the first upper strap (218) relative to the second upper strap (220);
(ii) the second connector (230) is shaped to releasably attach to the first connector (228) and not to the third connector, and wherein the fourth connector is shaped to releasably attach to the third connector and not to the first connector (228); or
(iii) the lower strap (204, 206) includes a seat end (216) and a back end (224), wherein the lower connection region (212) extends between the seat end (216) and the back end (224), and wherein the first connector (228) is coupled to the lower connection region (212) via a guide (232), the guide (232) being configured to slide between the seat end (216) and the back end (224).

## Patentansprüche

1. Patiententransportsystem (202), umfassend:
eine Patiententransportvorrichtung (100), umfassend einen Sitzabschnitt (104) und einen Rückenabschnitt (106) zum Stützen des Patienten;
ein Patientenrückhaltesystem (200), das einen unteren Gurt (204, 206) und einen oberen Gurt (218, 220) mit einem vorderen Ende (222), das mit dem unteren Gurt (204, 206) gekoppelt ist, und einem hinteren Ende (224) umfasst; und
ein Kopplungssystem (250), um eine lösbare Anbringung des Patientenrückhaltesystems (200) an der Patiententransportvorrichtung (100) zu ermöglichen, wobei das Kopplungssystem (250) umfasst:
einen Riegel (254), der mit dem hinteren Ende (224) des oberen Gurts (218, 220) gekoppelt ist,
eine Kopplungshalterung (252), die zur entfernbaren Anbringung am Rückenabschnitt (106) der Patiententransportvorrichtung (100) eingerichtet ist, und
ein Schließstück (256), das betriebsmäßig an der Kopplungshalterung (252) angebracht ist und dazu eingerichtet ist, den Riegel (254) entfernbar aufzunehmen, um den oberen Gurt (218, 220) zusammen mit dem Riegel (254) relativ zur Kopplungshalterung (252) zu halten;
wobei die Kopplungshalterung (252) ein Inneres (258) definiert, wobei das Schließstück (256) einen Schlitz (260) definiert, der so geformt ist, dass er sich in das Innere (258) der Kopplungshalterung (252) erstreckt, und zur Aufnahme mindestens eines Abschnitts des Riegels (254) darin geformt ist, um den oberen Gurt (218, 220) zusammen mit dem Riegel (254) relativ zur Kopplungshalterung (252) zu halten.

2. Patiententransportsystem (202) nach Anspruch 1,
wobei der Schlitz (260) des Schließstücks (256) eine Anschlagfläche (262) definiert;
wobei der Riegel (254) eine Schiene (264) umfasst, die eine Rückhaltefläche (266) definiert, die angeordnet ist, um die Anschlagfläche (262) des Schlitzes (260) zu kontaktieren, um eine Bewegung des Riegels (254) relativ zum Schließstück (256) mindestens teilweise zu begrenzen.

3. Patiententransportsystem (202) nach Anspruch 2,
wobei das Kopplungssystem (250) ferner umfasst:
einen Anker (272), der betriebsmäßig an einem von der Patiententransportvorrichtung (100) und der Kopplungshalterung (252) angebracht ist; und
eine Aufnahme (274), die betriebsmäßig am anderen von der Patiententransportvorrichtung (100) und der Kopplungshalterung (252) angebracht ist, wobei die Aufnahme (274) dazu eingerichtet ist, den Anker (272) lösbar zu sichern, um eine entfernbare Anbringung des Patientenrückhaltesystems (200) am Rückenabschnitt (106) der Patiententransportvorrichtung (100) zu erleichtern.

4. Patiententransportsystem (202) nach Anspruch 3,
wobei das Kopplungssystem (250) betreibbar ist zwischen:
einer Eingriffskonfiguration (296), in der die Aufnahme (274) den Anker (272) sichert, um eine Bewegung der Kopplungshalterung (252) relativ zum Rückenabschnitt (106) mindestens teilweise zu begrenzen; und
einer Außer-Eingriff-Konfiguration (300), in der die Aufnahme (274) den Anker (272) freigibt, um das Entfernen der Kopplungshalterung (252) vom Rückenabschnitt (106) zu erleichtern.

5. Patiententransportsystem (202) nach Anspruch 4,
wobei das Kopplungssystem (250) ferner umfasst:
eine Verriegelung des oberen Gurts (268), die zwischen dem Schließstück (256) und dem Riegel (254) angeordnet ist und selektiv betreibbar ist zwischen:
einer Rückhaltekonfiguration (270), um eine Bewegung des Riegels (254) relativ zum Schließstück (256) mindestens teilweise zu begrenzen; und
einer Freigabekonfiguration (276), um eine Bewegung des Riegels (254) relativ zum Schließstück (256) zuzulassen;
wobei das Kopplungssystem (250) optional daran gehindert wird, sich während des Betriebs der Verriegelung des oberen Gurts (268) in der Rückhaltekonfiguration (270) von der Eingriffskonfiguration (296) in die Außer-Eingriff-Konfiguration (300) zu bewegen.

6. Patiententransportsystem (202) nach Anspruch 5,
wobei die Verriegelung des oberen Gurts (268) eine Blockierlasche (316) umfasst, die betriebsmäßig an der Schiene (264) des Riegels (254) angebracht ist; und
wobei sich die Blockierlasche (316) in der Rückhaltekonfiguration (270) optional in die Aufnahme (274) erstreckt; und
wobei die Blockierlasche (316) in der Rückhaltekonfiguration (270) optional benachbart zum Anker (272) ist und das Kopplungssystem (250) daran hindert, sich von der Eingriffskonfiguration (296) in die Außer-Eingriff-Konfiguration (300) zu bewegen.

7. Patiententransportsystem (202) nach einem der Ansprüche 3 bis 6,
wobei der Anker (272) eine Ankerbasis (278), einen Ankerkopf (280) und einen Ankerschaft (282) umfasst, der sich zwischen der Ankerbasis (278) und dem Ankerkopf (280) erstreckt; und
wobei die Aufnahme (274) einen Aufnahmekörper (284) umfasst, der eine Aufnahmeöffnung (286) definiert, die einen ersten Öffnungsbereich (288), der geformt ist, um den Ankerkopf (280) dort hindurch aufzunehmen, und einen zweiten Öffnungsbereich (290) aufweist, der in Verbindung mit dem ersten Öffnungsbereich (288) angeordnet und geformt ist, um den Ankerschaft (282) darin aufzunehmen; und
wobei optional die Aufnahme (274) ferner eine Lasche (292) umfasst, die so geformt ist, dass sie sich in die Aufnahmeöffnung (286) erstreckt, und benachbart zum zweiten Öffnungsbereich (290) angeordnet ist, um eine Bewegung der Kopplungshalterung (252) zwischen dem ersten Öffnungsbereich (288) und dem zweiten Öffnungsbereich (290) mindestens teilweise einzuschränken.

8. Patiententransportsystem (202) nach einem der Ansprüche 3 bis 7,
wobei der untere Gurt (204, 206) ein Sitzende (216) und ein hinteres Ende (224) umfasst;
wobei die Kopplungshalterung (252) ferner als eine Rückenabschnitt-Kopplungshalterung (252) definiert ist, die mit dem hinteren Ende (224) des unteren Gurts (204, 206) gekoppelt ist;
wobei der Anker (272) ferner als ein Rückenabschnitt-Anker (272) definiert ist, der betriebsmäßig am Rückenabschnitt (106) der Patiententransportvorrichtung (100) angebracht ist;
wobei die Aufnahme (274) ferner als eine Rückenabschnitt-Aufnahme (274) definiert ist, die betriebsmäßig an der Rückenabschnitt-Kopplungshalterung (252) angebracht ist; und
wobei das Kopplungssystem (250) ferner umfasst:
einen Sitzabschnitt-Anker (312), der betriebsmäßig am Sitzabschnitt (104) der Patiententransportvorrichtung (100) angebracht ist, und
eine Sitzabschnitt-Aufnahme (314), die geformt ist, um den Sitzabschnitt-Anker (312) lösbar zu sichern.

9. Patiententransportsystem (202) nach Anspruch 8,
wobei die Rückenabschnitt-Kopplungshalterung (252) ferner eine Gurtbefestigung (306) umfasst, die dazu eingerichtet ist, das hintere Ende (224) des unteren Gurts (204, 206) aufzunehmen.

10. Patiententransportsystem (202) nach einem der Ansprüche 8 bis 9,
wobei das Kopplungssystem (250) eine Sitzabschnitt-Kopplungshalterung (308) umfasst, die mit dem Sitzende (216) des unteren Gurts (204, 206) gekoppelt ist, wobei die Sitzabschnitt-Aufnahme (314) betriebsmäßig an der Sitzabschnitt-Kopplungshalterung (308) angebracht ist.

11. Patiententransportsystem (202) nach Anspruch 10,
wobei der Rückenabschnitt (106) der Patiententransportvorrichtung (100) ferner eine Rückenabschnittsöffnung umfasst, die geformt ist, um die Rückenabschnitt-Kopplungshalterung (252) dort hindurch aufzunehmen und zu verhindern, dass die Sitzabschnitt-Kopplungshalterung (308) dort hindurch aufgenommen wird; und
wobei der Sitzabschnitt (104) der Patiententransportvorrichtung (100) ferner eine Sitzabschnittsöffnung (322, 324) umfasst, die geformt ist, um die Sitzabschnitt-Kopplungshalterung (308) dort hindurch aufzunehmen und zu verhindern, dass die Rückenabschnitt-Kopplungshalterung (252) dort hindurch aufgenommen wird.

12. Patiententransportsystem (202) nach Anspruch 11,
(i) wobei die Rückenabschnittsöffnung ein Rückenabschnittsöffnungsprofil (330) definiert, das geformt ist, um zuzulassen, dass die Rückenabschnitt-Kopplungshalterung (252) durch die Rückenabschnittsöffnung gelangt, und um zu verhindern, dass die Sitzabschnitt-Kopplungshalterung (308) durch die Rückenabschnittsöffnung gelangt; und
wobei die Sitzabschnittsöffnung (322, 324) ein Sitzabschnittsöffnungsprofil (332) definiert, das geformt ist, um zuzulassen, dass die Sitzabschnitt-Kopplungshalterung (308) durch die Sitzabschnittsöffnung (322, 324) gelangt, und um zu verhindern, dass die Rückenabschnitt-Kopplungshalterung (252) durch die Sitzabschnittsöffnung (322, 324) gelangt; oder
(ii) wobei die Rückenabschnitt-Kopplungshalterung (252) ein Rückenabschnitt-Kopplungshalterungsprofil (334) definiert;
wobei die Rückenabschnittsöffnung ein Rückenabschnittsöffnungsprofil (330) definiert;
wobei die Sitzabschnitt-Kopplungshalterung (308) ein Sitzabschnitt-Kopplungshalterungsprofil (336) definiert;
wobei die Sitzabschnittsöffnung (322, 324) ein Sitzabschnittsöffnungsprofil (332) definiert;
wobei eine Querschnittsfläche des Rückenabschnitt-Kopplungshalterungsprofils (334) in mindestens einer Abmessung größer ist als eine Querschnittsfläche des Sitzabschnittsöffnungsprofils (332); und
wobei eine Querschnittsfläche des Sitzabschnitt-Kopplungshalterungsprofils (336) in mindestens einer Abmessung größer ist als eine Querschnittsfläche des Rückenabschnittsöffnungsprofils (330).

13. Patiententransportsystem (202) nach Anspruch 11 oder 12,
wobei die Rückenabschnitt-Kopplungshalterung (252) ein Rückenabschnitt-Kopplungshalterungsprofil (334) definiert, das eine Rückenabschnitt-Kopplungshalterungslänge (338) und eine Rückenabschnitt-Kopplungshalterungsbreite (340) umfasst;
wobei die Rückenabschnittsöffnung ein Rückenabschnittsöffnungsprofil (330) definiert, das eine Rückenabschnittsöffnungslänge (342) und eine Rückenabschnittsöffnungsbreite (344) umfasst;
wobei die Sitzabschnitt-Kopplungshalterung (308) ein Sitzabschnitt-Kopplungshalterungsprofil (336) definiert, das eine Sitzabschnitt-Kopplungshalterungslänge (346) und eine Sitzabschnitt-Kopplungshalterungsbreite (348) umfasst;
wobei die Sitzabschnittsöffnung (322, 324) ein Sitzabschnittsöffnungsprofil (332) definiert, das eine Sitzabschnittsöffnungslänge (350) und eine Sitzabschnittsöffnungsbreite (352) umfasst;
wobei die Rückenabschnitt-Kopplungshalterungslänge (338) kleiner ist als die Rückenabschnittsöffnungslänge (342) und die Rückenabschnitt-Kopplungshalterungsbreite (340) kleiner ist als die Rückenabschnittsöffnungsbreite (344); und
wobei die Sitzabschnitt-Kopplungshalterungslänge kleiner ist als die Sitzabschnittsöffnungslänge (350) und die Sitzabschnitt-Kopplungshalterungsbreite kleiner ist als die Sitzabschnittsöffnungsbreite (352); und
wobei optional mindestens eine der folgenden Bedingungen erfüllt ist:
(i) die Rückenabschnitt-Kopplungshalterungslänge (338) ist größer als die Sitzabschnittsöffnungslänge (350);
(ii) die Rückenabschnitt-Kopplungshalterungsbreite (340) ist größer als die Sitzabschnittsöffnungsbreite (352);
(iii) die Sitzabschnitt-Kopplungshalterungslänge ist größer als die Rückenabschnittsöffnungslänge (342);
(iv) die Sitzabschnitt-Kopplungshalterungsbreite ist größer als die Rückenabschnittsöffnungsbreite (344).

14. Patiententransportsystem (202) nach einem der vorhergehenden Ansprüche,
wobei der obere Gurt (218, 220) einen Schulterbereich (226) umfasst, der sich zwischen dem vorderen Ende (222) und dem hinteren Ende (224) erstreckt; und
wobei die Patiententransportvorrichtung (100) ferner einen Gurtversteller (318, 320) umfasst, der betriebsmäßig am Rückenabschnitt (106) angebracht und geformt ist, um das hintere Ende (224) des oberen Gurts (218, 220) dort hindurch aufzunehmen, wobei der Gurtversteller (318, 320) dazu eingerichtet ist, lösbar in den Schulterbereich (226) des oberen Gurts (218, 220) einzugreifen, um die Spannung im oberen Gurt (218, 220) zwischen dem Gurtversteller (318, 320) und dem unteren Gurt (204, 206) einzustellen.

15. Patiententransportsystem (202) nach einem der vorhergehenden Ansprüche,
wobei der untere Gurt (204, 206) ferner als ein erster unterer Gurt (204) definiert ist, wobei der obere Gurt (218, 220) ferner als ein erster oberer Gurt (218) definiert ist, und wobei das Patientenrückhaltesystem (200) ferner einen zweiten unteren Gurt (206) und einen zweiten oberen Gurt (220) umfasst, und
wobei optional mindestens eine der folgenden Bedingungen erfüllt ist:
(i) jeder von dem ersten unteren Gurt (204) und dem zweiten unteren Gurt (206) weist einen Oberschenkelbereich (208), einen Taillenbereich (210) und einen unteren Verbindungsbereich auf, der zwischen dem Oberschenkelbereich (208) und dem Taillenbereich (210) angeordnet ist;
wobei jeder von dem ersten oberen Gurt (218) und dem zweiten oberen Gurt (220) einen oberen Verbindungsbereich (212) aufweist, der zwischen dem vorderen Ende (222) und dem hinteren Ende (224) angeordnet ist; und
wobei das Patientenrückhaltesystem (200) ferner umfasst:
einen ersten Verbinder (228), der mit dem unteren Verbindungsbereich (212) des ersten unteren Gurts (204) gekoppelt ist;
einen zweiten Verbinder (230), der mit dem unteren Verbindungsbereich (212) des zweiten unteren Gurts (206) gekoppelt ist und dazu eingerichtet ist, lösbar am ersten Verbinder (228) angebracht zu werden, um eine Bewegung des ersten unteren Gurts (204) relativ zum zweiten unteren Gurt (206) mindestens teilweise zu begrenzen;
einen dritten Verbinder, der mit dem oberen Verbindungsbereich (212) des ersten oberen Gurts (218) gekoppelt ist; und
einen vierten Verbinder, der mit dem oberen Verbindungsbereich (212) des zweiten oberen Gurts (220) gekoppelt ist und dazu eingerichtet ist, lösbar am dritten Verbinder angebracht zu werden, um eine Bewegung des ersten oberen Gurts (218) relativ zum zweiten oberen Gurt (220) mindestens teilweise zu begrenzen;
(ii) der zweite Verbinder (230) ist geformt, um lösbar am ersten Verbinder (228) angebracht zu werden und nicht am dritten Verbinder, und wobei der vierte Verbinder geformt ist, um lösbar am dritten Verbinder angebracht zu werden und nicht am ersten Verbinder (228); oder
(iii) der untere Gurt (204, 206) umfasst ein Sitzende (216) und ein hinteres Ende (224), wobei sich der untere Verbindungsbereich (212) zwischen dem Sitzende (216) und dem hinteren Ende (224) erstreckt, und wobei der erste Verbinder (228) über eine Führung (232) mit dem unteren Verbindungsbereich (212) gekoppelt ist, wobei die Führung (232) dazu eingerichtet ist, zwischen dem Sitzende (216) und dem hinteren Ende (224) zu gleiten.

## Revendications

1. Système de transport de patient (202) comprenant:
un appareil de transport de patient (100) comprenant une partie siège (104) et une partie dossier (106) pour soutenir le patient; et
un système de retenue de patient (200) comprenant une sangle inférieure (204, 206), une sangle supérieure (218, 220) ayant une extrémité avant (222) couplée à la sangle inférieure (204, 206) et une extrémité arrière (224), et
un système d'accouplement (250) pour faciliter la fixation amovible du système de retenue du patient (200) à l'appareil de transport du patient (100), le système d'accouplement (250) comprenant:
un mécanisme de verrouillage (254) couplé à l'extrémité arrière (224) de la sangle supérieure (218, 220),
une attache (252) conçue pour être fixée de manière amovible à la partie dossier (106) de l'appareil de transport de patients (100), et
un dispositif de retenue (256) fixé de manière fonctionnelle à l'attache (252) et conçu pour recevoir de manière amovible le mécanisme de verrouillage (254) afin de maintenir la sangle supérieure (218, 220) avec le mécanisme de verrouillage (254) par rapport à l'attache (252);
dans lequel l'attache (252) forme un espace intérieur (258), le dispositif de retenue (256) formant une fente (260) s'étendant dans l'espace intérieur (258) de l'attache (252) et conçue pour recevoir au moins une partie du mécanisme de verrouillage (254) en son sein afin de retenir la sangle supérieure (218, 220) avec le mécanisme de verrouillage (254) par rapport à l'attache (252).

2. Système de transport de patient (202) selon la revendication 1,
dans lequel la fente (260) du dispositif de retenue (256) forme une surface de butée (262); et
dans lequel le mécanisme de verrouillage (254) comprend une piste (264) définissant une face de retenue (266) agencée pour entrer en contact avec la surface de butée (262) de la fente (260) afin de limiter au moins en partie le mouvement du mécanisme de verrouillage (254) par rapport au dispositif de retenue (256).

3. Système de transport de patient (202) selon la revendication 2,
dans lequel le système d'accouplement (250) comprend en outre:
un système d'ancrage (272) fixée de manière fonctionnelle à l'un parmi l'appareil de transport de patient (100) et l'attache (252); et
un dispositif de réception (274) fixé de manière fonctionnelle à l'autre élément parmi l'appareil de transport de patient (100) et l'attache (252), le dispositif de réception(274) étant conçu pour fixer de manière amovible le système d'ancrage (272) afin de faciliter la fixation amovible du système de retenue de patient (200) à la partie dossier (106) de l'appareil de transport de patient (100).

4. Système de transport de patient (202) selon la revendication 3,
dans lequel le système d'accouplement (250) peut être utilisé selon:
une configuration de mise en prise (296) où le dispositif de réception (274) attache solidement le système d'ancrage (272) afin de limiter au moins partiellement le mouvement de l'attache (252) par rapport à la partie dossier (106); et
une configuration désolidarisée (300) où le dispositif de réception (274) libère le système d'ancrage(272) afin de faciliter le retrait de l'attache (252) de la partie dossier (106).

5. Système de transport de patient (202) selon la revendication 4,
dans lequel le système d'accouplement (250) comprend en outre:
un verrou de sangle supérieure (268) disposé entre le dispositif de retenue (256) et le mécanisme de verrouillage (254) et pouvant être actionné de manière sélective entre:
une configuration de retenue (270) pour limiter au moins partiellement le mouvement du mécanisme de verrouillage (254) par rapport au dispositif de retenue (256); et
une configuration libérée (276) pour permettre le mouvement du mécanisme de verrouillage (254) par rapport au dispositif de retenue (256); éventuellement
dans lequel le système d'accouplement (250) ne peut pas commuter de la configuration de mise en prise (296) à la configuration désolidarisée (300) pendant le fonctionnement du verrou de sangle supérieure (268) dans la configuration de retenue (270).

6. Système de transport de patient (202) selon la revendication 5,
dans lequel le verrou de sangle supérieure (268) comprend une languette de blocage (316) fixée de manière fonctionnelle à la glissière (264) du mécanisme de verrouillage (254); et, éventuellement,
dans lequel la languette de blocage (316) s'étend dans le dispositif de réception (274) dans la configuration de retenue (270); et éventuellement,
dans lequel la languette de blocage (316) est adjacente au système d'ancrage (272) dans la configuration de retenue (270) et empêche le système d'accouplement (250) de commuter de la configuration de mise en prise (296) à la configuration désolidarisée (300).

7. Système de transport de patient (202) selon l'une quelconque des revendications 3 à 6,
dans lequel le système d'ancrage (272) comprend une base d'ancrage (278), une tête d'ancrage (280) et une tige d'ancrage (282) s'étendant entre la base d'ancrage (278) et la tête d'ancrage; et
dans lequel le dispositif de réception (274) comprend un corps de réception (284) formant une ouverture de réception (286) comportant une première zone d'ouverture (288) formée pour y recevoir la tête d'ancrage (280), et comportant une deuxième zone d'ouverture (290) en communication avec la première zone d'ouverture (288) et formée pour y recevoir la tige d'ancrage (282); et éventuellement,
dans lequel le dispositif de réception (274) comprend en outre une languette (292) s'étendant dans l'ouverture du dispositif de réception (286) et disposée à proximité de la deuxième zone d'ouverture (290) afin de restreindre au moins en partie le mouvement de l'attache (252) entre la première zone d'ouverture (288) et la deuxième zone d'ouverture (290).

8. Système de transport de patient (202) selon l'une quelconque des revendications 3 à 7,
dans lequel la sangle inférieure (204, 206) comprend une extrémité d'assise (216) et une extrémité de dossier (224);
dans lequel l'attache (252) est en outre définie comme une attache de partie dossier (252) couplée à l'extrémité arrière (224) de la sangle inférieure (204, 206);
dans lequel le système d'ancrage (272) se présente aussi sous la forme d'un système d'ancrage de partie dossier (272) fixé de manière fonctionnelle à la partie dossier (106) de l'appareil de transport de patient (100);
dans lequel le dispositif de réception (274) se présente aussi sous la forme d'un dispositif de réception de partie dossier (274) fixé de manière opérationnelle à l'attache de partie dossier (252); et
dans lequel le système d'accouplement (250) comprend en outre:
un système d'ancrage de partie siège (312) fixé de manière fonctionnelle à la partie siège (104) de l'appareil de transport de patient (100), et
un dispositif de réception de partie siège (314) formé pour fixer solidement de manière amovible le système d'ancrage de partie siège (312).

9. Système de transport de patient (202) selon la revendication 8,
dans lequel le support d'accouplement de partie dossier (252) comprend en outre un support de sangle (306) conçu pour recevoir l'extrémité arrière (224) de la sangle inférieure (204, 206).

10. Système de transport de patient (202) selon l'une quelconque des revendications 8 à 9,
dans lequel le système d'accouplement (250) comprend un support d'accouplement de partie siège (308) couplé à l'extrémité de siège (216) de la sangle inférieure (204, 206), le dispositif de réception de partie siège (314) étant fixé de manière opérationnelle au support d'accouplement de partie siège (308).

11. Système de transport de patient (202) selon la revendication 10,
dans lequel la partie dossier (106) de l'appareil de transport de patient (100) comprend en outre une ouverture de partie dossier formée pour recevoir le support d'accouplement de partie dossier (252) à travers celle-ci et pour empêcher la réception du support d'accouplement de section de siège (308) à travers celle-ci; et
dans lequel la partie siège (104) de l'appareil de transport de patient (100) comprend en outre une ouverture de partie siège (322, 324) ménagée pour y recevoir le support d'accouplement de partie siège (308) et pour y empêcher la réception du support d'accouplement de partie dossier (252).

12. Système de transport de patient (202) selon la revendication 11,
(i) dans lequel l'ouverture de la partie dossier forme un profil d'ouverture de partie dossier (330) formé de manière à permettre au support d'accouplement de la partie dossier (252) de passer à travers l'ouverture de la partie dossier et à empêcher le support d'accouplement de la section de siège (308) de passer à travers l'ouverture de la partie dossier; et
dans lequel l'ouverture de la partie siège (322, 324) définit un profil d'ouverture de la partie siège (332) formé de manière à permettre au support d'accouplement de la partie siège (308) de passer à travers l'ouverture de la partie siège (322, 324) et à empêcher le support d'accouplement de la partie dossier (252) de passer à travers l'ouverture de la partie siège (322, 324); ou
(ii) dans lequel le support d'accouplement de la partie dossier (252) définit un profil de support d'accouplement de la partie dossier (334);
dans lequel l'ouverture de la partie dossier définit un profil d'ouverture de partie dossier (330);
dans lequel le support d'accouplement de la partie siège (308) définit un profil de support d'accouplement de la partie siège (336);
dans lequel l'ouverture de partie siège (322, 324) définit un profil d'ouverture de la partie siège (332);
dans lequel la section transversale du profilé de support d'accouplement de la partie dossier (334) est plus grande dans au moins une dimension que la section transversale du profilé d'ouverture de la partie siège (332); et
dans lequel la section transversale du profilé de fixation (336) de la partie siège est plus grande, dans au moins une dimension, que la section transversale du profilé d'ouverture (330) de la partie dossier.

13. Système de transport de patient (202) selon la revendication 11 ou 12,
dans lequel le support d'accouplement de la partie dossier (252) définit un profil de support d'accouplement de la partie dossier (334) comprenant une longueur de support d'accouplement de la partie dossier (338) et une largeur de support d'accouplement de la partie dossier (340);
dans lequel l'ouverture de la partie dossier définit un profil d'ouverture de la partie dossier (330) comprenant une longueur d'ouverture de la partie dossier (342) et une largeur d'ouverture de la partie dossier (344);
dans lequel le support d'accouplement de partie siège (308) définit un profil de support d'accouplement de partie siège (336) comprenant une longueur de support d'accouplement de partie siège (346) et une largeur de support d'accouplement de partie siège (348);
dans lequel l'ouverture de la partie siège (322, 324) définit un profil d'ouverture de la partie siège (332) comprenant une longueur d'ouverture de la partie siège (350) et une largeur d'ouverture de la partie siège (352);
dans lequel la longueur du support d'accouplement de la partie dossier (338) est inférieure à la longueur de l'ouverture de la partie dossier (342) et la largeur du support d'accouplement de la partie dossier (340) est inférieure à la largeur de l'ouverture de la partie dossier (344); et
dans lequel la longueur du support d'accouplement de la partie siège est inférieure à la longueur de l'ouverture de la partie siège (350) et la largeur du support d'accouplement de la partie siège est inférieure à la largeur de l'ouverture de la partie siège (352); et éventuellement,
dans lequel au moins l'une des conditions suivantes est remplie:
(i) la longueur du support d'accouplement de la partie dossier (338) est supérieure à la longueur d'ouverture de partie siège (350);
(ii) la largeur du support d'accouplement de la partie dossier (340) est supérieure à la largeur de l'ouverture de la section siège (352);
(iii) la longueur du support d'accouplement de la partie siège est supérieure à la longueur de l'ouverture de la partie dossier (342);
(iv) la largeur du support d'accouplement de la partie siège est supérieure à la largeur de l'ouverture de la partie dossier (344).

14. Système de transport de patient (202) selon l'une quelconque des revendications précédentes,
dans lequel la sangle supérieure (218, 220) comprend une zone épaule (226) s'étendant entre l'extrémité avant (222) et l'extrémité arrière (224); et
dans lequel l'appareil de transport de patient (100) comprend en outre un dispositif de réglage de sangle (318, 320) fixé de manière fonctionnelle à la partie dossier (106) et formé pour y recevoir l'extrémité arrière (224) de la sangle supérieure (218, 220), le dispositif de réglage de sangle (318, 320) étant conçu pour entrer en prise de manière amovible avec la zone d'épaule (226) de la sangle supérieure (218, 220) afin de régler la tension de la sangle supérieure (218, 220) entre le dispositif de réglage de sangle (318, 320) et la sangle inférieure (204, 206).

15. Système de transport de patient (202) selon l'une quelconque des revendications précédentes,
dans lequel la sangle inférieure (204, 206) est en outre définie comme une première sangle inférieure (204), dans lequel la sangle supérieure (218, 220) est en outre définie comme une première sangle supérieure (218), et dans lequel le système de retenue de patient (200) comprend en outre une deuxième sangle inférieure (206) et une deuxième sangle supérieure (220), et, éventuellement,
dans lequel au moins l'une des conditions suivantes est remplie:
(i) chacune des première sangle inférieure (204) et deuxième sangle inférieure (206) comporte une zone cuisse (208), une zone taille (210) et une zone de liaison inférieure disposée entre la zone cuisse (208) et la zone taille (210);
dans lequel chacune des première sangle supérieure (218) et deuxième sangle supérieure (220) comporte une zone de liaison supérieure (212) disposée entre l'extrémité avant (222) et l'extrémité arrière (224); et
dans lequel le système de retenue de patient (200) comprend en outre:
un premier connecteur (228) couplé à la zone de liaison inférieure (212) de la première sangle inférieure (204);
un deuxième connecteur (230) couplé à la zone de liaison inférieure (212) de la deuxième sangle inférieure (206) et conçu pour se solidariser de manière amovible au premier connecteur (228) afin de limiter au moins en partie le mouvement de la première sangle inférieure (204) par rapport à la deuxième sangle inférieure (206);
un troisième connecteur couplé à la zone de liaison supérieure (212) de la première sangle supérieure (218); et
un quatrième connecteur couplé à la zone de liaison supérieure (212) de la deuxième sangle supérieure (220) et conçu pour se solidariser de manière amovible au troisième connecteur afin de limiter au moins en partie le mouvement de la première sangle supérieure (218) par rapport à la deuxième sangle supérieure (220);
(ii) le deuxième connecteur (230) est conçu pour se solidariser de manière amovible au premier connecteur (228) et non au troisième connecteur, et dans lequel le quatrième connecteur est conçu pour se solidariser de manière amovible au troisième connecteur et non au premier connecteur (228); ou
(iii) la sangle inférieure (204, 206) comprend une extrémité de siège (216) et une extrémité de dossier (224), la zone de liaison inférieure (212) s'étendant entre l'extrémité de siège (216) et l'extrémité de dossier (224), et le premier connecteur (228) est couplé à la zone de liaison inférieure (212) par le biais d'un guide (232), le guide (232) étant conçu pour coulisser entre l'extrémité de siège (216) et l'extrémité de dossier (224).
